# EUROPEAN PATENT APPLICATION

(11) **EP 4 523 731 A1**
(43) Date of publication of application: **19.03.2025**
(21) Application number: 23197897.4
(22) Date of filing: 18.09.2023
(51) Int. Cl.: A61N 1/05, A61N 1/36, A61B 5/00

(54) **NEURAL INTERFACE AND CLOSED-LOOP DECODING AND NEUROMODULATION/NEUROSTIMULATION SYSTEM INCLUDING THE SAME**

(71) Applicant: INBRAIN Neuroelectronics SL, 08028 Barcelona (ES)
(72) Inventor: Aguilar, Carolina, 1168 Villars sous Yens (CH); Bakker, Bert, 4261 LH Wijk en Aalburg (NL); Garrido Ariza, José Antonio, 08960 Sant Just Desvern, Barcelona (ES); Decré, Michel, 5656 AE Eindhoven (NL)
(74) Representative: DTS Patent- und Rechtsanwälte PartmbB

(57) **Abstract**

The present invention provides a cortical or subcortical neural interface (100, 200) for delivering neuromodulation/neurostimulation to brain structures of a patient (P)and/or sensing brain signals of said patient, comprising a support (10, 20), e.g. a thin film, including an electrode array (12, 22) comprising a plurality of electrodes (14, 24) made of reduced graphene oxide (rGO), each electrode (14, 24) of said plurality of electrodes (14, 24) having an area in the micrometer range. The invention further provides a closed-loop system (300) for delivery of neuromodulation/neurostimulation to brain structures of a patient (P), the system (300) including the cortical or subcortical neural interface described above.

## Description

The present invention belongs to the technical field of neural decoding and delivery of neuromodulation/neurostimulation to brain structures of a patient, especially to mitigate symptoms of neurological disorders such as Parkinson's Disease (PD), and/or psychiatric disorder such as Obsessive Compulsive Disorders (OCD).

In particular, the present invention aims at providing a solution in potential replacement of Deep Brain Stimulation (DBS).

The invention provides an enhanced neural interface for delivering neuromodulation/neurostimulation to brain structures of a patient and/or decoding brain signals of said patient, e.g. a PD patient.

Also, the present invention provides an improved closed-loop system for delivering neuromodulation/neurostimulation to brain structures of a patient, e.g. a PD patient.

PD is a neurodegenerative disease characterized by the loss of dopaminergic neurons within the substantia nigra of the brain.

Clinically, the disease manifests mainly with motor impairments, including rigidity, tremor, bradykinesia and postural instability, although non-motor symptoms can lead to significant morbidity.

Large epidemiologic studies revealed around 10 million people with PD worldwide, affecting all races and cultures **[1].**

The age of onset is usually over 60, but it is estimated that one in 10 people are diagnosed before the age of 50, with slightly more men than women affected.

Neurological disorders such as PD are characterized by structural and functional abnormalities in multiple brain areas involving several distinct brain systems.

These abnormalities can be referred to as brain neuronal network deficiencies (BNND), which can broadly vary in causes, type of dysfunction, and clinical manifestation, e.g. existence of deleterious networks in the brain in case of neurological and psychiatric disorders, such as Alzheimer disease, Parkinson's disease (PD), Huntington's chorea, depression and/or schizophrenia. Network abnormalities are also described in patients diagnosed with, or with high-risk of, schizophrenia, attention deficit hyperactivity disorder, bipolar disorder, and/or OCD **[61].**

In PD, changes observed in the dynamic functional connectivity are suggestive of a reduction in functional segregation among brain networks and are correlated with the clinical severity of PD symptoms.

Additionally, a higher variability in the network global efficiency is observed, suggesting an abnormal global integration of the brain networks **[2].**

Deleterious neural networks are formed based on intimate interactions among key pathogenic factors. These deleterious networks could be the common pathway of these brain disorders and, when triggered by aging, genetic and environmental factors, may lead to a cascade of pathological alterations that characterize the illnesses.

Disorders induced by BNND may explain many neuropsychiatric and neurodegenerative diseases but are far away from being completely understood.

Treatments approaching these conditions from a brain network perspective offer a promising alternative to single-target pharmacological therapies that, so far, have largely proven insufficient **[3], [4].**

Some neuromodulation therapies, now approved by the USA Food and Drug Administration (FDA) and currently applied in clinical routine, have proven able to impact dysfunctional networks, providing successful therapeutic outcomes.

Presently, in PD, the use of local brain stimulation has proven effective in reducing the pathological symptoms in most patients.

However, clinical benefits with Standard of Care (SOC) devices are not without limits, and show large patient-to-patient variability. Several reasons may explain these outcomes, but it is hypothesized that the most relevant limiting factors derive from the fact that, in these therapies, stimulation is delivered with limited selectivity to the basal ganglia, without considering the effect of that therapeutic stimulation on brain networks affected by the disease in a particular patient.

Following an initial diagnosis, PD subjects are typically treated with Levodopa (a precursor of dopamine), with dopamine agonists or monoamine oxidase-B inhibitors. These represent different classes of drugs, and are all characterized by different benefit-risk profiles. Subjects who use these medications over a long term may experience development of drug-induced side effects including motor fluctuations, dyskinesia, or psychiatric complications **[5],** usually three to five years after starting of the medication.

However, it must be noted that those complications may be also due to disease progression.

The term dyskinesia describes involuntary, erratic, writhing movements of the face, arms, legs, and/or trunk, which usually occur one to two hours after a dose of medication has been absorbed into the bloodstream and is thus having its peak clinical effect.

For many patients, drug therapy alone is not effective anymore in controlling the symptoms of PD.

Over time, DBS has emerged as an option for PD patients to be an adjunct to those medications and provide additional benefit.

DBS may target either the subthalamic nucleus (STN) or the internal globus pallidus (GPi).

DBS is a technological evolution of the irreversible surgical ablation of the basal ganglia, originally used to treat some PD-affected patients.

In particular, instead of permanently damaging some target brain areas, DBS utilizes electrodes implanted in the same areas to interfere with pathological signals and thereby improve movements.

Once a patient is selected as a candidate for DBS, generally imaging is performed in order to determine the target site for lead placement.

STN is chosen as the target for treatment of most PD patients. However, GPi may be a better option in some other cases, depending on the observed symptoms.

Off-period motor symptoms and tremor improve in both targets.

Advantages of targeting STN target include more medication reduction, less frequent IPG changes, and a more favorable economic profile.

On the other hand, advantages of targeting GPi include more robust dyskinesia suppression, easier programming, and greater flexibility in adjusting medications **[6].**

Once the target is chosen, SOC surgical stereotactic techniques are used to implant the lead at the target site. Here, a frame or a robotic arm is used to control the insertion angle and depth. Surgery is planned with a preoperative image with fiducial markers and dedicated image processing.

Current DBS surgery involves two different surgical steps, i.e. lead implant and neurostimulator. The leads are placed through burr holes in the skull of the patient and connected to extensions that are tunneled down to a pacemaker-like implantable pulse generator (IPG) that is generally placed in the chest below the clavicle.

All of the existing approved DBS systems for this patient population use this basic setup, all involving a fully implanted system.

DBS with commercially-available leads is typically delivered by using a set-up including from 4 to 8 metal electrodes delivering energy.

The selection is based on localization within the target area and, more importantly, on the capacity to induce a therapeutic effect.

Usually, PD patients who are selected for DBS have already attempted one or more pharmacological therapies in order to control their disease, which have proven not completely effective.

DBS was established to be an efficient therapy for the treatment of PD symptoms, in particular with documented improvements in motor control, functionality, and quality of life.

Moreover, DBS allows on average to reduce Levodopa dose treatment, which may lead to lower Levodopa-related adverse effects and higher compliance **[7].**

Although STN DBS and Levodopa independently lessened motor severity in PD patients to a similar magnitude, their combined effect has proven greater than either treatment alone, thereby suggesting therapeutic synergism **[8].**

For this reason, DBS efficacy is generally assessed in off-medication conditions to evaluate effects of DBS, and in combination with antiparkinsonian drugs to assess real-life patients benefits.

DBS benefits on motor symptoms are most commonly evaluated using both subjective assessment from users and objective assessment by clinicians, e.g. neurologists, using Unified Parkinson's Disease Rating Scale (UPDRS), and more particularly the third part of the scale evaluating motor functions, rather than reporting on individual motor signs. Measurement of DBS benefits in PD patients has been established by several class I studies, e.g. in a randomized double-blind design with sham control clinical trial, as discussed in Vitek et al. **[9]** (the clinical trial being also known under the name of INTREPID).

In the context of this clinical trial, PD patients were implanted with a DBS system according to SOC, and were randomly assigned in a 3:1 ratio to receive either active therapeutic stimulation settings (active group) or subtherapeutic stimulation settings (control group) for a 3-month blinded period. During the blinded period, patients were not allowed to increase their antiparkinsonian medications per study protocol **[9].**

The primary efficacy outcome of the study was the difference in mean change from baseline visit to 3 months post-randomization between the active and control groups in the mean number of waking hours per day with good symptom control and no troublesome dyskinesias, as recorded by the user in a dedicated diary, with no increase in anti-parkinsonian medications **[9].**

The study reported that baseline duration for the active group was 7.78 hours, which increased to 12.37 hours after 12 weeks (3.74 hours improvement on average) **[9].**

Considering that patients were reporting to sleep 7.2 hours per day on average preoperatively, further considering that DBS therapy allows for about 1 more hour of sleep **[10],** the total awake time after 12 weeks of treatment for active group was estimated around 15.8 hours on average, leading to a ceiling improvement from baseline of time with good symptoms control of about 8 hours **[9].**

Otherwise stated, the 3.74 hours change from baseline reported for active group after 12 weeks of DBS use was less than half of the maximum possible improvement.

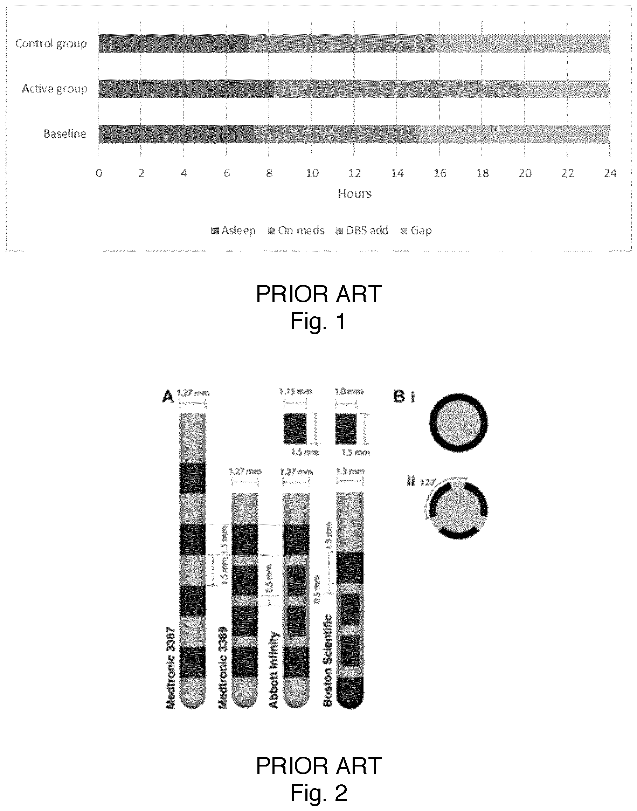

The results of the clinical trial discussed in Vitek et al. **[9]** are resumed in **Fig. 1****.**

UPDRS III scores were assessed in on- and off-medication conditions at baseline and at 12 weeks for both active and control groups. Change from baseline comparison between groups was performed as secondary endpoint, and showed significant reduction of UPDRS III score in on-stim/off-meds condition for active group, concluded to be clinically meaningful.

However, no significant reduction was observed in on-stim/on-meds condition.

This could be explained by the fact that individual motor scores for tremor (61 %), rigidity (51 %), bradykinesia (32%), and gait (39%) were all improved in the on-stim/off-meds, while only tremor and rigidity were significantly better in the on-stim/on-meds condition **[9].**

These results emphasize the importance of antiparkinsonian medication in symptoms management, and also confirm the role of DBS as an adjunctive therapy for PD treatment, supporting effects of medications on tremor and rigidity, as other symptoms are better managed by medication.

From a safety perspective, DBS surgery is overall assumed as having a reasonable risk profile.

The above-mentioned INTREPID clinical trial discussed in Vitek et al. **[9],** for example, reported 74 serious adverse events among 177 involved participants. Infection has been the most commonly reported serious adverse event associated with device-hardware/procedure (8 events, representing 2.7% of subjects). There were three events (each) of device-hardware/procedure-related serious adverse events of peri-operative intracranial hemorrhage (representing 1% of subjects) and seizure (representing 1% of subjects). After 12 weeks of use, 3 more cerebral hemorrhages and 6 more device related infections were reported as non-serious events.

Other meta-analysis and reviews on large cohorts of patients implanted with similar DBS systems (although not all suffering from PD) demonstrated similar outcomes **[11], [12],** as shown in **Table 1.**

It is noticeable that the rate of infections, surgical site complications, and device complications are lower in the most recent reports, showing improvements in the surgical procedure and devices over time.

Still, more than 10% device complication rate is reported in Rasiah et al. **[12],** suggesting that there is room for further improvement of device safety and reliability.

It is indeed a higher revision rate when compared, for example, to established head-mounted implanted active stimulator such as cochlear implants, showing device failure rate below 5% in adults and children, with a cumulative revision rate for primary implants at all ages increased linearly by 1% per year **[13].**

The difference in device complication rate between the results shown in **Table 1** and the outcome reported in the context of the INTREPID clinical trial **[9]** may also be reasonably explained in the light of the short follow-up period for the INTREPID clinical trial (only amounting in 12 weeks).

Adverse events related to stimulation are much more frequent.

In fact, the INTREPID clinical trial **[9]** reported a high incidence of 53.6% stimulation-related adverse events, most frequently involving dyskinesia (16,5%), balance disorders (5%), and dysphagia (3.3%).

On the other hand, the results reported in Appleby et al. **[11],** referring to a clinical trial carried out on a large cohort with longer follow-up time, showed a lower rate of 26.9% for stimulation-related adverse events, most frequently involving speech disturbance (4.8%) and delirium/confusion (4.1%).

Overall, the above-discussed DBS-related events and side effects support that there is room for further improvement.

Currently, DBS is delivered through leads using metallic electrodes having a relatively large area between 4 and 6 mm², with a limited number of electrodes, usually 4 to 8 electrodes.

Examples of DBS leads that are presently approved and used clinical treatment of PD symptoms are shown in **Fig. 2** (starting from the left: Medtronic 3387, Medtronic 3389, 8-electrode Abbott Infinity^{®} directional lead, and 8-electrodes Boston Scientific directional lead Vercise Cartesia^{®}).

The examples shown in **Fig. 2** represent the most commonly used leads in present DBS treatments, which have been used since DBS was first approved approximatively 30 years ago.

The prior art leads shown in **Fig. 2** include a quite limited number of electrodes (namely, 4 to 8 electrodes), each having a quite large area in the millimetric range (namely, between 1.5 and 6 mm²).

In both the 8-electrodes leads (Abbott Infinity^{®} directional lead, Boston Scientific Vercise Cartesia^{®}), the two middle electrodes are segmented into three smaller electrodes (**Fig. 2**).

This segmented electrode design allows to achieve a degree of directionality.

However, the overall area of the electrodes still remains quite large (**Fig. 2**), therefore allowing limited resolution.

The relatively large electrode areas, along with the reduced number of electrodes provided on each lead (i.e. 4 to 8 electrodes), represent a significant limitation.

Indeed, in the current clinical approaches using known lead designs, e.g. as shown in **Fig. 2****,** the subthalamic nucleus is generally assumed as the therapeutic target, while no directions regarding the specific placement of the lead within the STN are considered.

However, treating the STN as a homogenous entity means failing to understand its complex organization.

To get more details on STN organization, using a structural connectivity-based parcellation protocol on 7 T MRI diffusion-weighted images of 17 patients with PD scheduled for DBS surgery, the connections of the STN to the motor, limbic, and associative cortical areas were used to map the individual subdivisions of the nucleus **[14].**

The results revealed that the motor area of the STN is located in a dorso-lateral portion of the STN and occupies roughly 1/3 of the entire STN volume and less than half (approx. 2 to 4 mm) of the length of the STN along a normal lead trajectory based on beta-band local field potentials (LFPs) **[14].**

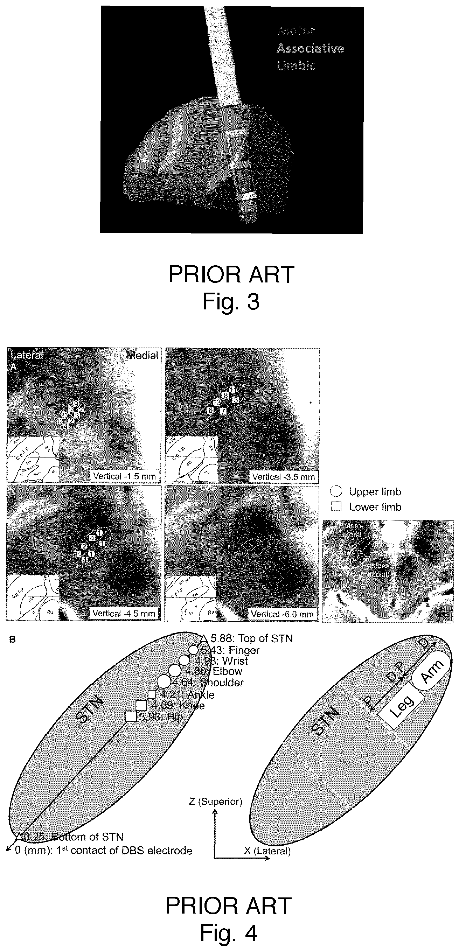

This area is therefore of a similar length to a single known DBS electrode, typically between 1 to 2 mm (**Fig. 3**).

However, even such a small area of tissue has been shown to possess its own complex somatotopic organization, where controls of extremities, trunk and face are segregated in different smaller areas.

In Sasaki et al. **[15],** the somatotopic organization within the STN from 77 surgical cases was examined using microelectrode recording, while performing passive movements of each joint to capture single neuronal activities and identify movement-related cells.

The study revealed that most movement-related cells were distributed in the upper third of the STN in its superior, lateral, and posterior regions as shown in **Fig. 4** **[15].**

A recent study discussed in Rodriguez-Rojas et. al. **[16]** confirmed this somatotopic organization of the motor area of the STN, although using a different experimental method. Here, the authors assessed STN connectivity patterns by using functional-MRI diffusion-weighted imaging and analytical tractography to reconstruct structural cortico-subthalamic connectivity. A somatotopic representation was observed within the sensorimotor area of the STN as a projection of the somatotopy of the motor cortex.

The complexity of the anatomical structures within the basal ganglia requires precise delivery of stimulation.

In a modelling study discussed in Ineichen et al. **[17],** including conductivity, permittivity, and permeability of STN and of surrounding brain tissues, stimulation directionality patterns were found to be affected by tissue properties and electrode location, this having an inevitable impact on DBS therapeutic effects.

A poor therapeutic window (where for "therapeutic window" is intended to denote the difference in threshold between therapeutic effects and side effects) may result from non-optimal electrode placement. Surgical repositioning of the lead may be required to improve the therapeutic performance.

In other cases, a reduced therapeutic window may be the consequence of subject-specific anatomy. Especially in instances where there are anatomical considerations for a particular patient, existing approaches are usually unable to implement a proper adaption, in particular due to the size of the electrodes.

Stimulation of different areas of the STN may generate a better management of specific motor symptoms.

For example, tremor seems to be better controlled when leads are placed in the posterior part of the STN, while bradykinesia seems to be better controlled when leads are placed in a more anterior position.

As an example, the Medtronic 3389 lead, schematically illustrated in **Fig. 2****,** includes four relatively large ring electrodes (1.5 mm height x 1.27 mm diameter, with an overall area of 6 mm²) for delivery of stimulation to the brain and sensing local electrophysiological signals (LFPs).

The field of stimulation obtained by activating one of said four electrodes is approximately spherical around the lead. The generation of such an omnidirectional electric field, in combination with sub-optimal lead positioning, results in poor selectivity of the anatomical structures surrounding the lead that are being activated, leading to the activation of unwanted anatomical structures and generation of side effects.

For this reason, improved leads (so-called "directional" leads) have been designed containing up to 12metal electrodes (e.g., Medtronic SenSight^{®}, Abbott Infinity^{®}, Boston Scientific Vercise Cartesia^{®}, or directional Aleva^{®}), where two ring electrodes are divided into three independent segments, each having an area of about 1-2 mm², allowing to achieve a certain degree of directional selectivity.

However, there is room for further improvement.

The above-mentioned limitations of existing DBS leads regarding stimulation delivery, analogously apply when brain signals recording is concerned.

In particular, DBS leads according to the prior art fail to accurately record and sense signals within such a small anatomical area, even more if the same electrodes are used for both stimulation and recording.

In fact, such leads are limited in a way that monopolar or bipolar recording configurations only can sample large areas of tissue, with potential confounding signals coming not only from the motor area of the STN, but also from outside the margins of the entire STN (including cortical volume conducted signals).

On the other hand, the potential to reduce electrode size in those known DBS leads is limited by the materials used.

In particular, Platinum (Pt) and platinum-iridium (Pt-Ir) constrain geometrical dimensions due to impedance limits.

Otherwise stated, significantly smaller metal electrodes could potentially be manufactured, but this would result in high impedance, with consequent increase in energy requirements and safety concerns of charge injection for stimulation, further than increased noise in the recorded signals.

Summarizing, the quite reduced number of electrodes provided on each lead, the relatively large dimensions of the electrodes and the used materials, such Platinum (Pt) or platinum-iridium (Pt-Ir), represent a major limitation in the perspective of obtaining high stimulation selectivity as well as the ability to record and decode brain signals with high spatial and signal resolution.

Also, known DBS systems are mostly adapted to deliver open-loop stimulation, this meaning that stimulation is delivered continuously (or at best according to a specific pre-established duty cycle), with no real-time adjustment of the stimulation parameters, and not specificity related to individual patient's biomarkers.

This approach shows several drawbacks, such as tolerability-related issues with respect to potential side effects, reduction of the therapeutic effect over time, and battery depletion.

Moreover, it must be considered that symptomatic activity usually varies over time, e.g. during the day or over multiple days.

This is explained in a study described in Powers et al. **[18]** using a smartwatch-based motor symptoms detector for 6 months on a large cohort of (non-implanted) PD patients.

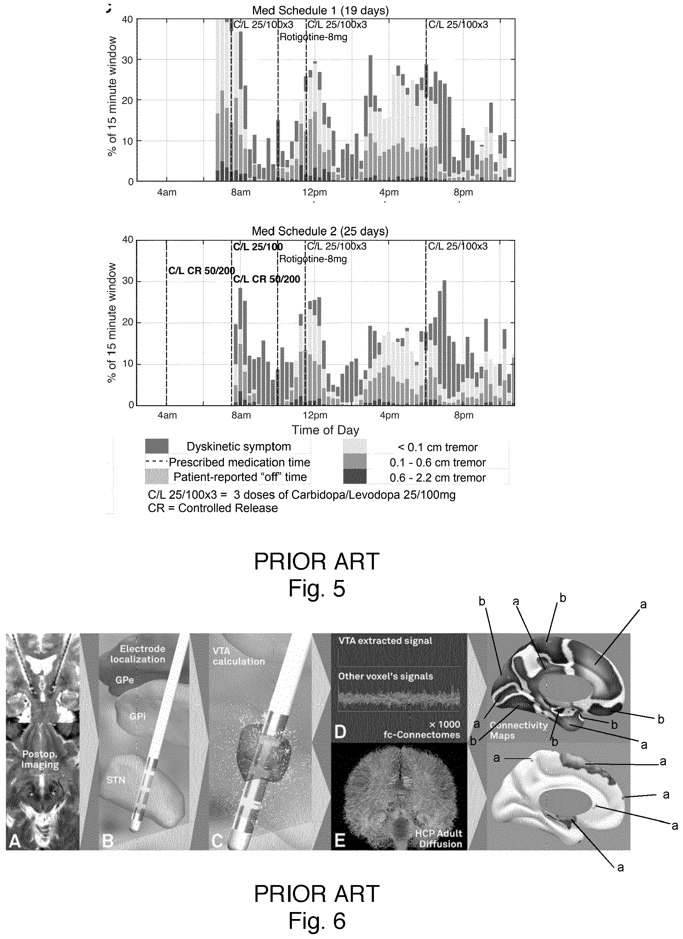

Here, large fluctuations of dyskinesia and tremor and effects of medications were observed over a day in all patients with variability across patients, and within patients from one day to another, as schematically illustrated in **Fig. 5****.**

Apparently, these therapeutic needs that vary over time cannot be taken into account when an open-loop approach is implemented.

DBS seems to extend the number of hours with good symptoms control without the need of medications increase. However, it is important to consider that current evaluation criteria still show some limitations.

Firstly, as all reported durations are made by patients in a diary, it is difficult to precisely determine their level of accuracy.

Moreover, such reporting does not give information on the moments of the day when the patient is actually not experiencing pathological symptoms. In other words, it cannot be evaluated whether the 4 hours during which the patient is experiencing poor symptoms control occur all at once or in different moments over the day, and if this happens always at the same time during the day, following some temporal patterns, or randomly.

Objective measurement of pathologic activity (e.g. using neurophysiology or worn accelerometers), together with tracking of drug intake, could bring much more details and precision on the time of on- and off- therapy, as well as possible daily patterns.

Finally, reporting on a total number of hours per day does not give any qualitative information on those moments when patients are experiencing good or bad symptoms control, i.e. whether it is an activity or a rest moment.

At present, little is known about the detailed physiological mechanisms of neural stimulation of deep brain structures and the reason for its effectiveness.

Yet, some evidence could be found, highlighting the activation of brain networks following stimulation of deep brain structures and the therapeutic relevance of such activation observed at multiple brain levels.

Horn et. al. **[19]** evaluated the connectivity between the stimulation site and other brain regions in PD patients treated with DBS by identifying the structural and functional connectivity profile of effective DBS to the STN.

This evaluation was made by estimating, for each patient, the volume of tissue activated by the DBS electrode from individual postoperative images, and then applying a combination of normative and PD pathologic averaged brain connectome to estimate the brain networks activated from a given stimulated volume of tissue **[19].**

In particular, the approach described in Horn et. al. **[19]** included the following steps: acquiring pre-/postoperative imaging (**Fig. 6A**) localizing DBS electrodes in standard space (**Fig. 6B**), calculating the volume of tissue activated (VTA) based on stimulation parameters (**Fig. 6C**), and calculating functional (**Fig. 6D**) and structural (**Fig. 6E**) connectivity from the VTA to the rest of the brain using high-quality normative connectome data.

In the context of **Fig. 6****,** positive correlations are denoted with **(a)** whereas negative correlations (anticorrelations) are denoted with **(b).**

As a result, the authors observed that a specific pattern of structural and functional connectivity with STN DBS electrodes correlates with clinical outcome across patients in PD. Moreover, the authors could appreciate the prognostic property of this approach by successfully predicting outcomes in an independent cohort of DBS users **[19].**

These findings support the notion that targets of therapeutic brain stimulation may be brain networks, and not individual brain regions such as the STN or GPi.

Another study (described in Boutet et al. **[20]**), implemented by using functional Magnetic Response Imaging (fMRI) in response to DBS in PD patients, supports this notion and the importance of brain network activation to deep nucleus stimulation.

The primary purpose of this study **[20]** was to identify specific markers of optimal DBS using fMRI to ease device fitting (in particular, to select the optimal electrode and voltage level). The activation of ipsilateral primary motor cortex, ipsilateral thalamus, and contralateral cerebellum was identified as a maker of optimal STN DBS, suggesting a large brain network activation to STN DBS, further optimized with a control of activation of this network at different levels **[20].**

This evidence clearly points toward the importance of monitoring the brain network response at various relevant locations to evaluate the efficacy of stimulation and potentially adjust the delivered therapy accordingly.

In view of the above, it is an object of the present invention to provide a neural interface and a closed-loop system including the same, which allow delivering neuromodulation/neurostimulation to brain structures of a patient with high level of stimulation selectivity and recording/decoding brain signals with enhanced spatial and signal resolution.

This object is achieved by the provision of a neural interface as defined in claim 1 and a closed-loop system as defined in claim 8.

According to the invention, a neural interface for delivering neuromodulation/neurostimulation to brain structures of a patient and/or sensing brain signals of the patient is provided, which comprises at least:
a support, wherein said support includes and/or carries at least an electrode array,
the electrode array at least comprising a plurality of electrodes made of reduced graphene oxide (rGO), each electrode of said plurality of electrodes having an area in the micrometer range.

Also, according to the invention, a closed-loop system for delivery of neuromodulation/neurostimulation to brain structures of a patient comprises:
a stimulation unit comprising at least one neural interface as defined above, said at least one neural interface being configured and adapted to deliver neuromodulation/neurostimulation to brain structures of the patient;
a sensing unit including at least one neural interface as defined above, said at least one neural interface being configured and adapted to sense brain signals of the patient, and
a control unit, operatively connected to the stimulation unit and the sensing unit, the control unit being configured and adapted to perform real-time decoding of brain signals from the sensing unit and control one or more stimulation parameters for the stimulation unit based on said brain signals from the sensing unit.

The present invention provides a neural interface.

In particular, said neural interface can be used for delivering neuromodulation/neurostimulation to brain structures of a patient and/or sensing brain signals of a patient.

The same neural interface can be used for both delivering neuromodulation/neurostimulation and sensing brain signals.

The neural interface comprises a support.

For example, the said support may at least partially be and/or comprise a thin film and/or be made of a nanostructure and/or a microstructure.

In particular, the term thin film is to be understood as a stack of layers of materials ranging from fractions of a nanometer to several micrometers in thickness. Thin film layers can be obtained applying a variety of thin-film deposition/preparation techniques including sputtering, evaporation, chemical vapor deposition, molecular beam epitaxy, atomic layer deposition, spin coating, 3D printing, or the like. The stack of layers exhibits a variety of functionalities, such as conductive layers, insulating layers, passivating layers, or the like.

The support, e.g. a thin film, includes and/or carries an electrode array comprising a plurality of electrodes.

The electrodes are made of reduced graphene oxide (rGO).

Also, each electrode of said plurality of electrodes is characterized by an area in the micrometer range.

The invention is based on the basic idea that, by the provision of a neural interface comprising a dense arrangement of rGO electrodes with areas in the micrometer range, it is possible to deliver neuromodulation/neurostimulation with high level of stimulation selectivity and recording/decoding a broader neural signal bandwidth, enabling to sense biomarkers that metal electrodes according to the prior art cannot (or barely) detect. The use of rGO electrodes having areas down to the micrometer range allows stimulation to be directionally steered towards targeted areas, at the same time enabling 10 to 100 times higher spatial resolution and high signal-to-noise ratio, compared to the leads according to the prior art.

The use of rGO electrodes allows obtaining high charge injection, low electrical impedance (even though the electrode area is reduced), and high signal-to-noise ratio (allowing to sense high-frequency signals up to typical of single- and multi-unit activity).

In particular, the physical properties of graphene allow obtaining micrometer-range electrodes while maintaining low electrical impedance and high-charge injection capacity.

Also, thanks to the properties of rGO, the neural interface is capable of sensing signals with high signal-to-noise ratio and delivering stimulation within the STN with high spatial selectivity and low angular resolution (down to 50-60°).

Advantageously, the area of each electrode is comprised between 1.0 and 0.0005 mm².

Advantageously, the electrode array comprises at least 20 electrodes.

For example, the electrode array may include 40 electrodes.

In another example, the electrode array may include 50 electrodes.

In yet another example, the electrode array may include from 128 to 1024 electrodes.

As mentioned, the provision of micrometer-range rGO electrodes allows achieving precise stimulation while allowing decoding of neural signals with high signal-to-noise ratio, which cannot be obtained with the millimeter-range metal electrodes according to the prior art.

Advantageously, the electrode array may comprise a plurality of electrodes having different areas in the micrometer range.

For example, one or more electrodes having a smaller area can be used for sensing smaller areas targeting single- and multi-unit activity, optimizing SNR and enlarging bandwidth. On the other hand, one or more electrodes having a larger area can be used for a more extended activation area targeting larger groups of neurons and measure e.g. LFPs.

Also, electrodes having different areas can be selected depending on characteristics of the stimulation target, spatial selectivity, charge injection, current density, required compliance voltage, and/or impedance.

The neural interface may be configured and adapted to be implanted cortically.

Alternatively, the neural interface may be configured and adapted to be implanted subcortically.

Advantageously, the thin film is made of a flexible material.

Preferably, said flexible material may include metal or a metal alloy.

Additionally or alternatively, said flexible material may include a polymer material.

Conveniently, said polymer material may include one among Polylmide, Parylene, or Polydimethylsiloxane (PDMS).

The neural interface of the invention allows sensing a large variety of brain signals over time.

For instance, said brain signals may include brain oscillations (e.g., beta, gamma, HFO).

Additionally or alternatively, said brain signals may include evoked responses to subcortical stimulation.

These recordings are characterized by a high resolution, both spatially and temporally.

Spatial high resolution is obtained thought the provision of a quite large number of rGO electrodes, arranged to form a high-density neural interface allowing recording/sensing activity of different locations nearby the implantation site.

On the other hand, temporal high resolution is mainly enabled by the properties of graphene.

In particular, rGO electrodes in the micrometer scale having low impedance allow for a high signal-to-noise ratio, enabling the recording/sensing of local neural activity at both low and high frequencies (with supporting sensing electronics).

The invention further provides a closed-loop system for delivery of neuromodulation/neurostimulation to brain structures of a particular patient.

According to a non-limiting example, said patient can be a Parkinson's Disease (PD) subject.

The system includes a stimulation unit.

In particular, the stimulation unit comprises at least one neural interface as described above, said at least one neural interface being configured and adapted to deliver neuromodulation/neurostimulation to brain structures of the patient.

The system further comprises a sensing unit.

The sensing unit comprises at least one neural interface as described above, said at least one neural interface being configured and adapted to sense brain signals of the patient.

The same neural interface can be used for both delivering neuromodulation/neurostimulation and sensing/ brain signals of the patient.

Still further, the system comprises a control unit.

The control unit is operatively connected to the stimulation unit and the sensing unit.

In particular, the control unit is configured and adapted to perform real-time decoding of brain signals from the sensing unit and control one or more stimulation parameters for the stimulation unit based on said brain signals from the sensing unit.

The system allows obtaining improved decoding and modulation of impaired brain networks of the nigrostriatal pathways, in a closed-loop fashion.

The closed-loop system according to the invention is capable of bringing significant benefits to patients, e.g. PD patients, reducing multiple pathological symptoms in motor disorders conditions and allowing maintaining therapeutic achievements over time.

In particular, compared to known DBS systems, the system of the invention allows achieving:
- improved symptoms management (by the provision of selective stimulation patterns which may address particular symptoms with high level of precision);
- longer ON time without troublesome symptoms;
- reduced side effects;
- lower UPDRS scores;
- higher quality of life for the patient;
- optimization and potential reduction of concomitant medication (e.g., Levodopa);
- empowering of patients through the transmission of their own data to help managing the disease, and
- improvement of healthcare resources by follow up clinicians' notification for patients in need of immediate care through risk stratifications alerts.

The at least one neural interface of the stimulation unit may include a subcortical lead.

Additionally or alternatively, the at least one neural interface of the stimulation unit may include a cortical lead.

Advantageously, said subcortical and/or cortical lead may be configured and adapted to deliver selective stimulation to basal ganglia to elicit a therapeutic response, based on individual patient's brain signals.

Stimulation is carried out with high-resolution, meaning that the target subcortical structure is stimulated at a very precise location (based on pathological biomarkers detection) more effectively and with limited adverse stimulation of surrounding, healthy structures.

This also allows reducing the variability of therapeutic effects by offering more stimulation pattern options.

Still further, side effects of stimulation are significantly reduced.

The sensing unit includes at least one cortical neural interface and/or at least one subcortical neural interface.

The brain signals may include at least one among brain oscillation (e.g. LFP) and/or evoked responses to subcortical stimulation.

Preferably, the sensing unit includes one cortical neural interface and one subcortical neural interface.

As mentioned, the same neural interface can be used for both delivering neuromodulation/neurostimulation and sensing brain signals of the patient.

Targeted, subcortical stimulation can be delivered through a subcortical neural interface based on a combination of decoded cortical brain signals and subcortical brain signals.

Advantageously, the control unit may be configured and adapted to implement a machine learning algorithm for recording and processing brain signals of the patient and generate stimulation patterns for the stimulation unit based on the processed brain signals.

Accordingly, it is possible to reliably monitor relevant symptomatic biomarkers from brain signals and adjust the delivered stimulation accordingly, thereby improving the therapeutic outcome.

Advantageously, the control unit may be further configured and adapted to:
process brain signals from the sensing unit and determine a physiological response of the patient to the delivered stimulation therapy;
map subcortical stimulation target points based on the determined physiological response of the patient, and
control the stimulation unit to deliver neuromodulation/neurostimulation based on the performed mapping.

This allows further optimizing the delivered stimulation according to the patient's needs.

The control unit may be implantable.

In particular, the control unit may be configured and adapted for implantation on the patient's skull.

The provision of an implantable control unit allows to reduce the overall dimensions of the system, further than improving the patient's comfort during use.

In this case, the system may further comprise an extra-corporeal power supply unit configured and adapted to wirelessly supply power to the implantable control unit.

In particular, the implantable control unit may include a battery that can be charged through an extra-corporeal power supply unit.

The system may further comprise a clinician computer.

For example, said clinician computer can be a portable device capable of implementing smart functionalities, e.g. a tablet computer or the like.

Alternatively, the clinician computer may be implemented by means of a web-based portal, e.g. accessing at least one server.

The clinician computer is configured and adapted to implement direct or indirect bi-directional communication with the control unit.

The clinician computer comprises a control means configured and adapted to run a dedicated software means to:
determine a patient's response to the delivered stimulation therapy based on detected brain signals from the sensing unit, and/or
provide an input signal to the control unit to adjust one or more stimulation or control loop parameters for the stimulation unit based on a determined patient's response to the delivered therapy and/or one or more predefined, patient-specific therapeutic targets.

This allows a clinician to monitor the patient's response to the delivered stimulation over time and, when appropriate, control the delivered treatment by adjusting one or more stimulation parameters according to the patient's response and/or specific therapeutic goals.

Advantageously, said software means of the clinician computer may be configured and adapted to implement web or cloud-based computing and/or machine learning algorithms and/or deep learning algorithms to:
analyze detected brain signals of the patient;
define the patient's response to the delivered therapy based on said detected brain signals, and
define optimized stimulation parameters based on the determined patient's response to the delivered stimulation therapy.

Preferably the patient's response to the delivered stimulation therapy is defined based on personalization algorithm suggestions, more preferably Al-based personalization algorithm suggestions.

This allows obtaining a highly-personalized, symptom-targeted stimulation therapy.

Also, this allow anticipating on the role of modulation versus pharmacology, so as to adapt stimulation to increase the therapeutic effect while further reducing medication.

The system implements high density decoding and modulation on its sensing unit , which renders almost impossible to program it with the current SOC programming methods, mostly due to the large number of combinations.

The complexity of electrode selection in multi-electrode leads can be reduced by using brain decoding-driven methods.

For instance, it is possible to sense LFPs neural signals and then down selecting electrode combinations based on somatotopic localization of signal biomarkers (e.g., beta band, spiking activity) **[21], [22].**

Also, more than one brain network can be activated by basal ganglia stimulation in relation to different symptoms **[23].**

The use of learning algorithms and/or deep learning algorithms plays a significant role in order to address complexity in personalization of the delivered stimulation therapy, since a large quantity of neurophysiological data can be considered.

Advantageously, the clinician computer may further comprise a display means.

In particular, said display means may be configured and adapted to display information regarding the detected brain signals, current stimulation parameters and/or other information on the delivered stimulation therapy.

Accordingly, the clinician is enabled to monitor the delivered stimulation therapy as well as the patient's response in a way that is intuitive and user-friendly.

Advantageously, said software means of the clinician computer may be further configured and adapted to:
collect and store data regarding patient's response to the delivered stimulation therapy for a plurality of patients subject to treatment, and
classify each of said patients based on a respective response to the delivered modulation therapy.

This enables the clinician to collect data regarding therapy response/effectiveness from all his/her patients at once, which facilitates patient pool follow-up.

Preferably, said software means is configured and adapted to classify patients according to a respective health-risk level.

In particular, patients may be classified according to the following ranking criteria:
low risk;
medium risk;
high risk.

This approach enables the clinician to prioritize follow-up appointments with patients showing outcomes that are poorer than expected or degradation of performance, for a programming adjustment and/or a new medication schedule.

This further allows to optimize hospital resources.

The system may further include a patient computer.

The patient computer is configured and adapted to implement direct or indirect bi-directional communication with the control unit.

The patient computer may comprise a control means configured and adapted to run a dedicated software means to:
receive a user input though a user input means of the patient computer, and
provide an input signal to the control unit to adjust one or more stimulation or control loop parameters for the stimulation unit, based on said user input.

The user input from the patient may include information concerning one or more among drug intake, how he/she is feeling, mood and/or energy level, such that the delivered therapy can be adjusted accordingly.

Preferably, said user input means includes a display means of the touch-screen type.

Additionally or alternatively, said user input means may be configured and adapted to receive a voice command and/or a user command provided by tapping on a predefined sensitive part.

This is particularly useful in case of patients affected by movement disorders, who are not always able to operate a mobile device such as a smartphone, a tablet computer, a smartwatch or the like.

The display means may as well be used to display relevant information for the patient regarding the delivered therapy.

Accordingly, the patient may be provided with a comprehensive overview on the ongoing stimulation therapy, which enables easily and intuitively monitoring therapeutic effects. The patient computer may be further configured and adapted to implement direct or indirect bi-directional communication with the clinician computer.

Advantageously, the control means of the patient computer may be further configured and adapted to:
receive a feedback from the patient regarding the delivered stimulation therapy,
provided through said user input means, and
forwarding the patient's feedback to the clinician computer in real time.

The patient's feedback may include information concerning one or more among drug intake, how he/she is feeling, mood and/or energy level.

Accordingly, the clinician may be promptly informed regarding therapy effectiveness, possible side effects, or the like. Based on the received patient's feedback, the clinician may consider whether stimulation parameters and/or drugs intake need being adjusted.

Also, the clinician may efficiently plan follow-up appointments at relevant times when it is most needed. The clinician may provide information to the patient though the clinician computer.

Information from the clinician may include, inter alia, risk-classification or the like.

In particular, in case the clinician computer is implemented by means of a web-based portal and the patient computer is connected to the web (e.g., through a mobile device), communication between the clinician computer and the patient computer can be implemented without the provision of any dedicated hardware means.

The patient computer may be a wearable device capable of implementing smart functionalities.

Preferably, the wearable device is provided with a sensor means that is configured and adapted to measure physiological data of the patient.

Preferably the physiological data of the patient include one or more among heart rate, blood pressure, body temperature and/or body motion/acceleration.

For example, the patient computer can be a smartwatch or the like, which can be easily carried an intuitively used by the patient.

For example, the one or more stimulation parameters may include one or more among stimulation frequency, stimulation amplitude, stimulation pulse-width, ratio (pulse-width and intensity) between cathodic and anodic pulses, duty cycling, burst, and/or electrode combination selection.

Further details and advantages of the present invention shall now be disclosed in connection with the drawings:
**Fig. 1** Diagram showing the results of the clinical trial carried out on a plurality of PD patients, discussed in Vitek et al. **[9]. Reproduced from Vitek et al. [9];**
**Fig. 2** Examples of DBS leads approved and currently used in DBS treatment of PD symptoms according to the prior art. **A.** (*from the left*) Medtronic 3387; Medtronic 3389; 8-electrode Abbott Infinity^{®} directional lead, and 8-electrode Boston Scientific directional lead Vercise Cartesia^{®} . **B.** Examples of known DBS electrodes in cross-section. **(i)** Axisymmetric ring electrodes (see Medtronic 3387 and Medtronic 3389) and other cylindrical electrodes on directional leads. **(ii)** Axisymmetric ring electrodes segmented into three smaller electrodes (8-electrode Abbott Infinity^{®} directional lead, 8-electrode Boston Scientific directional lead). **Reproduced from Anderson et.al. [27].** ;
**Fig. 3** Diagram providing a schematic representation of STN connectivity in a human subject, overlaid with an inserted subcortical DBS lead according to the prior art;
**Fig. 4** **A.** Plots of locations of movement-related cells responding to single joints in 3 direction coordinates relative to midcommisural point. Upper limb-related cells were significantly more commonly found in the lateral, anterior, and superior regions of the STN than in the lower limb-related cells. **B.** Plots of the average locations of movement-related cells responding to single joints and the corresponding DBS electrode in 3 direction coordinates. The coordinates and usage ratios of active electrodes are shown. Average location of active electrodes was slightly inferior, medial, and posterior to the sites of MRCs in the STN. **Reproduced from Sasaki et. al. (2019) [15].**
**Fig. 5** Smartwatch symptom profiles (% per 15-min window) aggregated across multiple days according to medication schedules for a given subject. **Upper plot:** Higher tremor levels were observed in the morning and mid-afternoon, which matched patient-reported "off" times. **Lower plot:** A reduction in the amount of tremor was seen during the patient's mid-afternoon "off' time on the new medication schedule (day 20 onward). **Reproduced from Powers et al. (2021) [18].**
**Fig. 6** Method for identifying DBS connectivity. Processing steps include: **A.** acquiring pre-/postoperative imaging; **B.** localizing DBS electrodes in standard space; **C.** calculating the volume of tissue activated (VTA) based on stimulation parameters; **D.** calculating functional and **E.** structural connectivity from the VTA to the rest of the brain using high-quality normative connectome data. The processing stream using the connectome datasets defined in healthy subjects is shown and was used in all primary analyses. **Reproduced from Horn et. al. (2017) [19].**

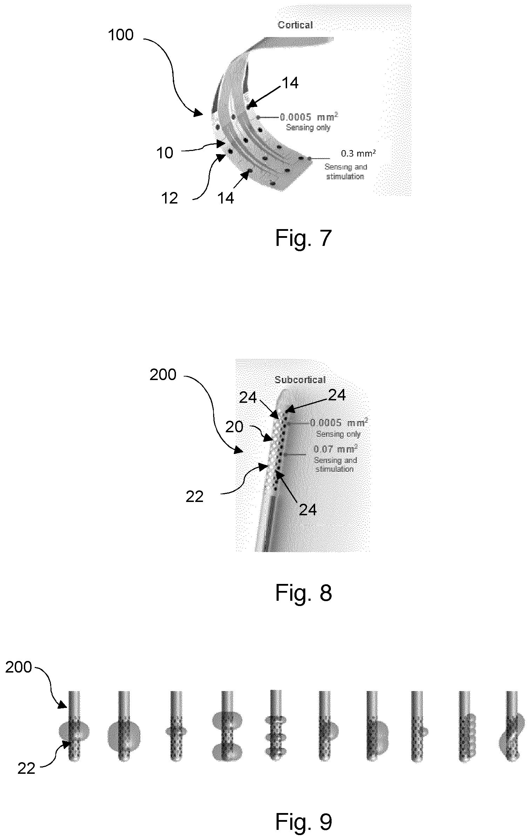

**Fig. 7** Schematic view of a configuration of a neural interface according to the invention. Here, the neural interface is configured to be implanted cortically.
**Fig. 8** Schematic view of an alternative configuration of the neural interface according to the invention. Here, the neural interface is in the shape of a lead configured to be implanted subcortically.
**Fig. 9** Diagram showing versatility of steering modulation in stimulation delivered using the neural interface (subcortical lead) of **Fig 8****.**

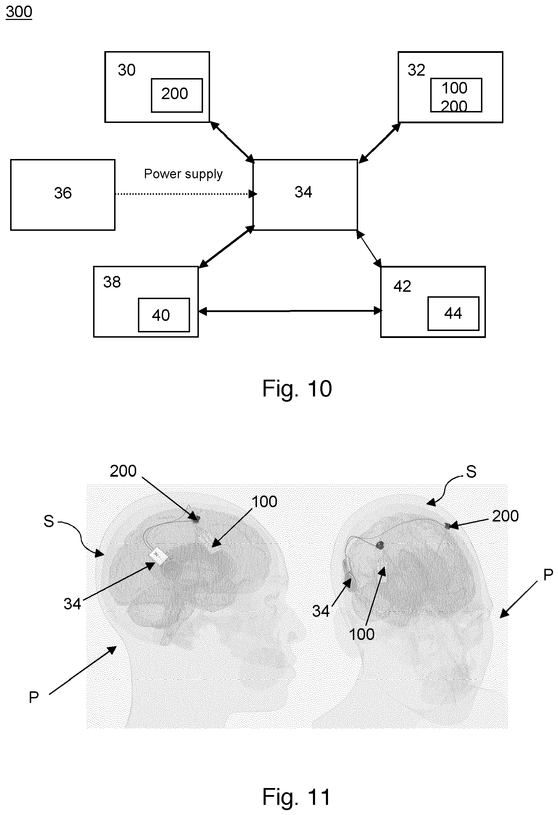

**Fig. 10** Diagram showing the components of a closed-loop system for delivery of neuromodulation/neurostimulation to a patient according to an embodiment of the invention, and how these components interact.
**Fig. 11** Diagrams showing different views of an exemplary arrangement of the implantable components of the system of **Fig. 10****,** in an implanted state.

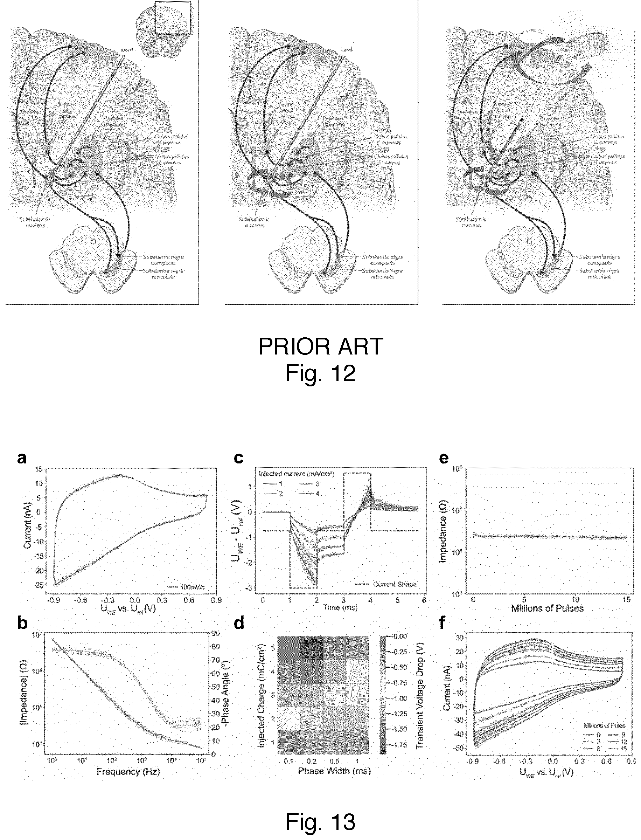

**Fig. 12** Diagram showing a schematic view of the functioning of an open-loop DBS system (**left panel**), a closed-loop system using subcortical LFP signals (**middle panel**), and a closed-loop system using a combination of subcortical and cortical recordings to regulate brain network response with subcortical high selectivity s**timulation** (**right panel**). **Reproduced from Okun [62].**
**Figs. 13a-f** Diagrams showing rGO electrochemical characterization. **(a)** Cyclic voltammetry at 100 mV/s of rGO microelectrodes. **(b)** Electrochemical impedance spectroscopy of rGO microelectrodes; it shows the module and phase of the impedance vs frequency. (c) Voltage response to current-controlled biphasic pulses of 1 ms/phase (dashed lined) applied through rGO electrodes, corresponding to charge injection values of up to 4 mC/cm². **(d)** Map of the cathodic voltage excursion occurred at the interface between rGO microelectrodes and the electrolyte during the injection of current pulses at different levels of injected charge and pulse widths. **(e)** Impedance at 1 kHz measured with rGO over continuous stimulation with biphasic pulses of 1 ms/phase. The injected charge for all the microelectrodes was 15 µA (3 mC/cm²). **(f)** Cyclic voltammetry (100 mV/s scan rate) measured periodically during continuous stimulation. **Reproduced from Viana et. al. [24].**

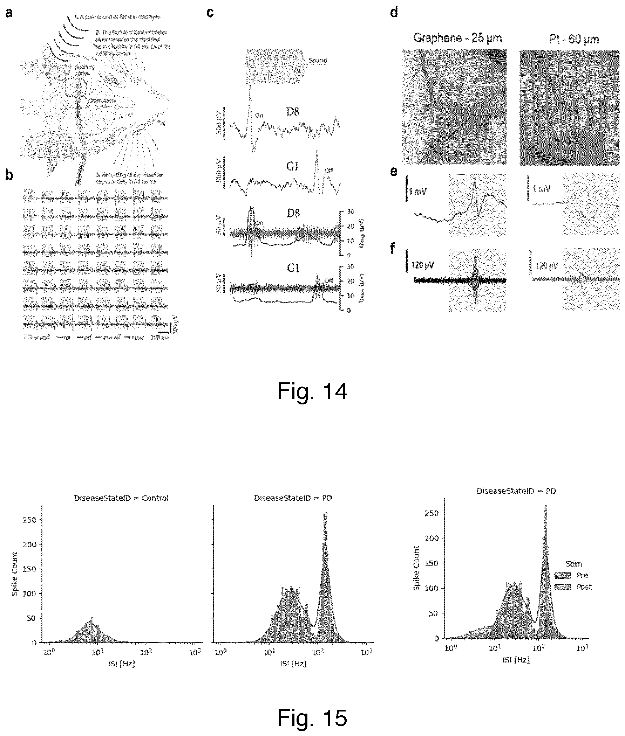

**Figs. 14a-f** Diagrams showing rGO sensing capabilities. **(a)** Schematic diagram of the acute experiment using a flexible thin film containing an array of rGO electrodes to sense epicortical neural activity in a rat. Evoked activity was induced by pure tones of 16 kHz and 200 ms duration (3 ms rise and 20 ms fall times) presented at 90 dB SPL to generate evoked activity. **(b)** Mapping of the evoked neural activity depicting a single event across all 64 rGO microelectrodes. Depending on the region, onset, offset, both, or no onset/offset responses are recorded. **(c)** Response of regions G1 (onset) and D8 (offset) to the sound stimulus (arrow on top). The bottom graphs represent the same signal high-pass filtered at 200 Hz to reveal high frequency multi-unit activity, confirmed by a simultaneous increase of the RMS value of the signal. **(d)** rGO array and Pt-array on the auditory cortex of a rat. (**e** and **f**) Representative raw traces of the evoked responses (**e)** and the same signal high-pass filtered at 200Hz **(f)** for rGO (left) and Pt (right) micro-electrodes. **Reproduced from Viana et. al. [24].**
**Fig. 15** Diagrams showing inter-spike frequency distribution of multi-unit activity measured by small rGO electrode in the STN in healthy control rodents (**left panel**), and in PD rodent model (**middle panel**). Left panel shows the inter-spike frequency distribution in PD rodent model before (pre) and after (post) therapeutic STN stimulation.

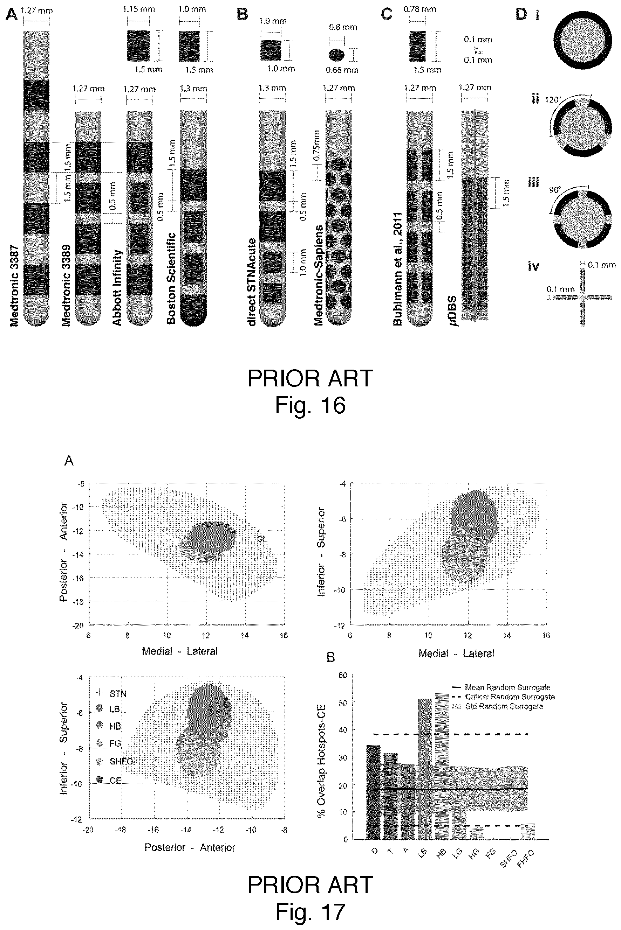

**Figs. 16A-D** Diagrams providing an overview of clinical DBS leads and emerging directional DBS technology. **A.** FDA-approved lead designs as shown in **Fig. 2****. B.** Additional directional leads that have not yet been approved by the FDA, but have been implanted in human patients for testing, including the 8-electrode direct STNAcute and the 40-electrode Medtronic-Sapiens lead. **C.** Additional lead designs that have not been implanted into human patients, including the 16-electrode segmented lead discussed in Buhlmann et. al. **[25]** and the micro-array based DBS (µDBS) with 1760 electrodes discussed in Willsie and Dorval **[26]. D.** Cross-sections of the DBS electrodes. **(i)** Axisymmetric ring electrodes, as seen in the Medtronic 3387 and Medtronic 3389 and other cylindrical electrodes on directional leads. **(ii)** Axisymmetric ring electrodes segmented into three smaller electrodes, as found on the 8-electrode Abbott Infinity^{®} directional lead, the 8-electrode Boston Scientific directional lead, and the direct STNAcute. **(iii)** Four electrodes per rotation can be found in the Medtronic-Sapiens and the segmented lead from Buhlmann et al. **[25]. (iv)** Plus-shaped cross-section found on the µDBS lead, with 32 electrodes per rotation. **Reproduced from Anderson et.al. [27].**
**Figs. 17A-B** Diagrams showing the overlap of clinical and spectral hot spots. **A.** 2D probabilistic representation of a selection of representative spectral, i.e., Low-Beta (LB), High-Beta (HB), FG, and Slow-HFO (SHFO) and Clinical Efficacy (CE) hot spots within the STN grid. In the top-right subplot depicting the inferior-superior and medial-lateral axes, the CE hot spot is covered by the spectral hot spots given its more posterior location. **B.** Bar plot showing the percentage overlap between the spectral hot spot and clinical hot spot. Black line, shaded area, and dotted line represent the mean, SD (standard deviation), and critical values (α = [0.025, 0.975]) of the random surrogate distribution. The LB and HB sub-bands share the largest volume with the clinical hot spot, and HG, FG, and SHFO share the lowest volume. **Reproduced from Averna et al. [29].**

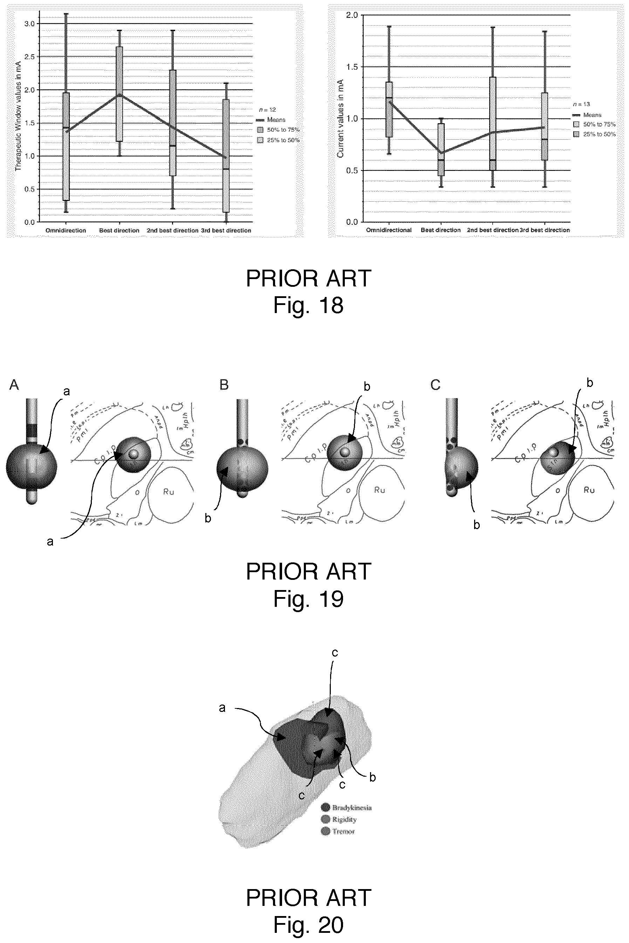

**Fig. 18** **Left panel:** box plot representing the width of the therapeutic window (percentiles 0, 25, 75, 100), for omnidirectional stimulation and for stimulation in each single direction. Thick line = mean therapeutic window. **Right panel:** Box plot of the electrical current producing a meaningful therapeutic effect (percentiles 0, 25, 75, 100), for omnidirectional stimulation and stimulation in each single direction. Thick line = mean current threshold. **Reproduced from Polio et al. [30].**
**Figs. 19A-C** In detail: **A.** Conventional deep brain stimulation lead with 4 cylindrical electrodes. Energizing of one electrode yields a spherical stimulation field **(a),** influencing both the target as the adjacent internal capsule (C.p.i.p.). **B.** 32-electrodes experimental lead. Activation of a circular array of 12 electrodes in 3 rows produces a spherical stimulation field **(b)** equivalent to conventional stimulation. **C.** Activation of an array of 4 electrodes yields a field of stimulation **(b),** which can be modulated to avoid stimulation of adjacent structures, only stimulating the target area. **Reproduced from Contarino et. al. [31].**
**Fig. 20** Diagram showing functional connectivity profiles predictive of motor improvement after subthalamotomy. Rendering of effective lesions inducing >65% improvement in bradykinesia **(a),** rigidity **(b),** and tremor **(c)** after subthalamotomy. **Reproduced from Rodriguez-Rojas et al. [16].**

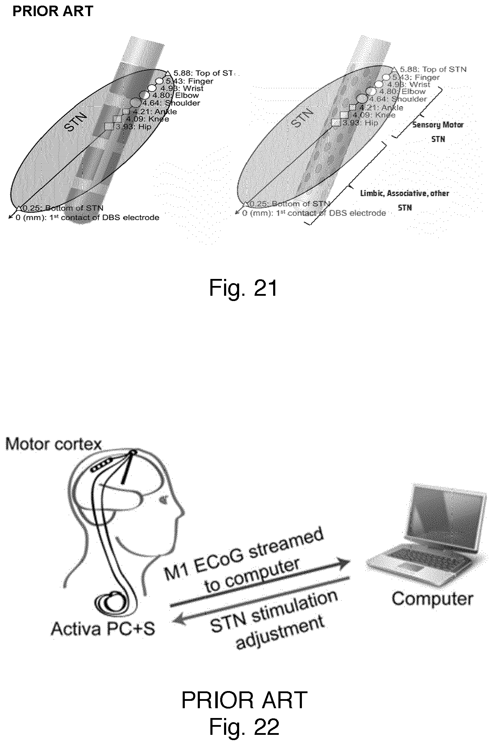

**Fig. 21** Diagram showing a schematic representation of STN somatotopy as schematized by Sasaki et al. **[15]** with overlapped illustration of a known subcortical Boston Scientific directional DBS lead, in an implanted state (**left panel**) and a subcortical neural interface (subcortical lead) according to the invention, in an implanted state (**right panel**).
**Fig. 22** Diagram showing a schematic illustration of adaptive DBS utilizing Activa PC+S under control of an external computer. **Reproduced from Swann et al. [32].**
**Figs. 7-8** provide schematic views of respective configurations of a neural interface 100, 200 for delivering neuromodulation/neurostimulation to brain structures of a patient P and/or sensing brain signals of a patient P according to an embodiment of the invention.

In a non-limiting example, the patient P may be a PD subject.

According to a further, non-limiting example, the patient P may be a subject affected by a psychiatric disorder, e.g. OCD.

The same neural interface 100, 200 can be used for both delivering neuromodulation/neurostimulation and sensing brain signals (**Figs. 7-8**).

In particular, **Fig. 7** shows a configuration of the neural interface 100 according to the invention, where said neural interface 100 is configured to be cortically implanted.

**Fig. 8** shows an alternative configuration of the neural interface 200 according to the invention, where said neural interface 200 is configured to be subcortically implanted.

In the shown embodiment (**Fig. 8**), said subcortical neural interface 200 is a subcortical lead configured to deliver neuromodulation/neurostimulation to brain structures of the patient P and/or sensing brain signals of the patient P.

The neural interface 100, 200 comprises a support 10, 20, including and/or carrying at least an electrode array 12, 22.

In the present embodiment, the support 10, 20 at least partially is and/or comprises a thin film and/or is made of a nanostructure and/or a microstructure.

In particular, **Figs. 7-8** show a configuration where the support 10, 20 is a thin film 10, 20.

In the present embodiment, said thin film 10, 20 is made of a flexible material.

Preferably, said flexible material includes metal or a metal alloy and/or a polymer material.

Preferably, said polymer material includes one among Polylmide, Parylene, or Polydimethylsiloxane (PDMS).

The electrodes 14, 24 are made of reduced graphene oxide (rGO).

Advantageously, each electrode 14, 24 of said plurality of electrodes 14, 24 has an area in the micrometer range.

The use of rGO electrodes 14, 24 having areas down to the micrometer range allows stimulation to be directionally steered towards targeted areas with a high level of selectivity (**Fig. 9**).

Also, this configuration enables 10 to 100 times higher spatial and signal sensing and stimulation resolution compared to the DBS leads according to the state of the art.

The neural interface 100, 200 according to the invention allows decoding biomarkers that known metal electrodes, e.g. Platinum (Pt) and platinum-iridium (Pt-Ir) electrodes, cannot (or can barely) detect.

The use of rGO allows obtaining high charge injection, low electrical impedance (even in case of electrodes having reduced dimensions) as well as high signal-to noise ratio (up to high frequencies typical of single- and multi-unit activity).

The physical properties of graphene allow the production of electrodes with a diameter in micrometer-range (such as electrodes 14, 24 shown in **Figs. 7-8**), while maintaining low electrical impedance and high-charge injection capacity.

Each electrode 14, 24 has an area ranging between 1.0 and 0.0005 mm².

In one example, the electrode array 12, 22 comprises at least 20 electrodes 14, 24.

In another example, the electrode array 12, 22 may include 50 electrodes 14, 24.

In yet another example, the electrode array 12, 22 may include from 128 to 1024 electrodes 14, 24.

For example, the electrode array 12, 22 may comprise 40 electrodes 14, 24.

In the shown embodiment, the electrodes 14, 24 forming the electrode array 12, 22 have different areas (**Figs. 7-8**).

For example, **Fig. 7** shows a configuration where the electrode array 12 comprises electrodes 14 having an area of 0.0005 mm² for sensing only, and electrodes 14 having an area of 0.3 mm² for both sensing and stimulation.

As a further example, **Fig. 8** shows a configuration where the electrode array 22 comprises electrodes 24 having an area of 0.0005 mm² for sensing only, and electrodes 24 having an area of 0.07 mm² for both sensing and stimulation.

Not shown is that the electrodes 14, 24 forming the array 12, 22 may have the same area.

The neural interface 100 can be configured and adapted to be implanted cortically (**Fig. 7**).

Alternatively, the neural interface 200 can be configured and adapted to be implanted subcortically (**Fig. 8**).

The invention further provides a closed-loop system 300 for delivery of neuromodulation/neurostimulation to brain structures of a patient P.

In a non-limiting example, the patient P may be a PD subject.

According to a further, non-limiting example, the patient P may be a subject affected by a psychiatric disorder, e.g. OCD.

**Fig. 10** schematically shows the different components of a closed-loop system 300 according to an embodiment of the invention and how these components interact.

The system 300 comprises a stimulation unit 30.

The stimulation unit 30 comprises at least one neural interface 100, 200 as described above.

In particular, said at least one neural interface 100, 200 is configured and adapted to deliver neuromodulation/neurostimulation to brain structures of the patient P.

The system 300 further comprises a sensing unit 32.

The sensing unit 32 comprises at least one neural interface 100, 200 as described above.

In particular, said at least one neural interface 100, 200 is configured and adapted to sense brain signals of the patient P.

Preferably, said brain signals of the patient P include at least one among brain oscillation and/or evoked responses to subcortical stimulation.

The same neural interface 100, 200 can be used for both delivering neuromodulation/neurostimulation and sensing brain signals of the patient P (**Figs. 7-8**).

Still further, the system 300 comprises a control unit 34.

The control unit 34 is operatively connected to the stimulation unit 30 and the sensing unit 32 (**Fig. 10**).

The control unit 34 is configured and adapted to perform real-time decoding of brain signals from the sensing unit 32 and control one or more stimulation parameters for the stimulation unit 30 based on said brain signals from the sensing unit 32.

In the present embodiment, said one or more stimulation parameters include at least one among stimulation frequency, stimulation amplitude, stimulation pulse-width, ratio (pulse-width and intensity) between cathodic and anodic pulses, duty cycling, burst, and/or electrode combination selection.

This combined approach integrating real-time brain activity monitoring and feedback allows enhancing precision and effectiveness of delivered neuromodulation/neurostimulation treatment.

In particular, the system 300 of the invention has proven effective in bringing significant benefits to patients, e.g. PD patients, reducing multiple symptoms in motor disorders conditions and allowing maintaining therapeutic achievements over time.

In the present embodiment, the at least one neural interface 200 of the stimulation unit comprises is a subcortical lead 200.

Not shown is that, additionally or alternatively, the at least one neural interface 100 of the stimulation unit may comprise a cortical lead 100.

In the present embodiment, said cortical and/or subcortical lead 100, 200 is configured and adapted to deliver selective stimulation to basal ganglia to elicit a therapeutic response.

The sensing unit 32 includes at least one cortical neural interface 100 and/or at least one subcortical neural interface 200.

In the present embodiment, the sensing unit 32 includes one cortical neural interface 100, e.g. as shown in **Fig. 7****,** and one subcortical neural interface 200, e.g. as shown in **Fig. 8****.**

In the present embodiment, the control unit 34 is an implantable control unit 34, in particular adapted for implantation on the skull S of the patient P.

Not shown is that the control unit 34 may be non-implantable.

An overview of the implantable components of the system 300 in an implanted stated is schematically provided in **Fig. 11****.**

There is also an extra-corporeal power supply unit 36, configured and adapted to wirelessly supply power to the control unit 34, while being implanted (as shown with dotted arrow in **Fig. 10**).

In the present embodiment, the control unit 34 is configured and adapted to implement a machine learning algorithm for recording and processing brain signals of the patient P and generate stimulation patterns for the stimulation unit 30 based on the processed brain signals.

Also, in the present embodiment, the control unit 34 is further configured and adapted to:
process brain signals from the sensing unit 32 and determine a physiological response of the patient P to the delivered stimulation therapy;
map subcortical stimulation target points based on the determined physiological response of the patient P, and
control the stimulation unit 30 to deliver neuromodulation/neurostimulation based on the performed mapping.

Advantageously, in the present embodiment, the system 300 further comprises a clinician computer 38.

The clinician computer 38 is configured and adapted to implement direct or indirect bi-directional communication with the control unit 34 (**Fig. 10**).

Not shown is that the clinician computer 38 comprises a control means.

In particular, said control means is configured and adapted to run a dedicated software means to:
determine a patient's response to the delivered stimulation therapy based on detected brain signals from the sensing unit 32, and/or
provide an input signal to the control unit 34 to adjust one or more stimulation or control loop parameters for the stimulation unit based on the determined patient's response to the delivered therapy and/or one or more predefined, patient-specific therapeutic targets.

In the present embodiment, said software means is further configured and adapted to implement web or cloud-based computing and/or machine learning algorithms and/or deep learning algorithms to:
analyze detected brain signals of the patient P;
define the patient's response to the delivered therapy based on said detected brain signals, and
define optimized stimulation parameters based on the determined patient's response to the delivered stimulation therapy.

Preferably, the patient's response to the delivered stimulation therapy is defined based on personalization algorithm suggestions, more preferably Al-based personalization algorithm suggestions.

Advantageously, in the present embodiment, the clinician computer 38 further comprises a display means 40 (**Fig. 10**).

In particular, said display means 40 of the clinician computer 38 is configured and adapted to display information regarding the detected brain signals, current stimulation parameters and/or other information on the delivered stimulation therapy.

Still further, the software means of the clinician computer 38 is configured and adapted to:
collect and store data regarding the patient's response to the delivered stimulation therapy for a plurality of patients P subject to treatment, and
classify each of said patients P based on a respective response to the delivered stimulation therapy.

In particular, patients P can be classified according to a respective health-risk level.

For instance, patents P may be classified according to the following ranking criteria:
low risk;
medium risk;
high risk.

In the present embodiment, the system 300 further comprises a patient computer 42.

The patient computer 42 is configured and adapted to implement direct or indirect bi-directional communication with the control unit 32 (**Fig. 10**).

The patient computer 42 comprises a control means configured and adapted to run a dedicated software means to:
receive a user input though a user input means of the patient computer 42, and
provide an input signal to the control unit 34 to adjust one or more stimulation or control loop parameters for the stimulation unit 30, based on said user input.

Said user input means may include display means 44 of the touch-screen type.

Additionally or alternatively, said user input means may be configured and adapted to receive a voice command and/or a user command provided by tapping on a predefined sensitive part.

The display means 44 may as well be used to display relevant information for the patient P regarding the delivered therapy.

Accordingly, the patient P may be provided with a comprehensive overview on the ongoing stimulation therapy, which enables easily and intuitively monitoring therapeutic effects.

Advantageously, the patient computer 42 is also configured and adapted to implement direct or indirect bi-directional communication with the clinician computer 38 (**Fig. 10**).

The control means of the patient computer 42 is configured and adapted to:
receive a feedback from the patient P regarding the delivered stimulation therapy,
provided through said user input means, and
forward the patient's feedback to the clinician computer 38 in real time.

Advantageously, the patient computer 42 can be a wearable device capable of implementing smart functionalities.

Preferably, said wearable device is provided with a sensor means (not shown) that is configured and adapted to measure physiological data of the patient.

Preferably, said physiological data of the patient include one or more among heart rate, blood pressure, body temperature and/or body motion/acceleration.

For example, the patient computer 42 can be a smartwatch or the like, capable of being easily carried and intuitively used by the patient P.

### Therapy phases

The therapy phases described below refer to a treatment performed using a preferred configuration of the system according to the invention on a PD subject whom chosen stimulation target is the STN.

### Patient selection

A PD subject may be referred for implantation if he/she is experiencing significant motor symptoms, such as tremors, stiffness, and difficulty with movement, despite treatment with medications. Implantation may also be considered when the subject is experiencing medication-related side effects that are affecting quality of life.

Implantation candidacy criteria may be based on an evaluation process including physical examination, imaging tests such as MRI or CT, neuropsychological testing, and multidisciplinary review.

In case the subject is deemed to be a good candidate for surgery, the implantation procedure is planned.

Patient selection process is similar to existing DBS referral process.

### Implantation

Preoperative imaging is necessary to plan the insertion of subcortical neural interfaces on STN targets and the position of the cortical neural interface on the motor cortex.

Then, the implanted components of the system are surgically placed in a single-step procedure.

In a preferred configuration, the system includes two subcortical neural interfaces and a single cortical neural interface.

The implantable control unit is secured on the skull, both subcortical neural interfaces are placed using state-of-the-art stereotactic surgery (e.g., by using a stereotactic robotic arm), and the cortical neural interface is positioned according to the planning.

A set of neurophysiology recordings can be performed perioperatively to evaluate the accuracy of neural interfaces positioning, taking advantage of the neurophysiology sesning capabilities of the system.

Firstly, all electrodes on the subcortical neural interfaces are used to automatically measure STN-specific neurophysiological signature (e.g., evoked response, beta activity, high-frequency activity) to evaluate the position of the interface related to STN.

This target response telemetry feature gives valuable information on neural interface placement as the percentage of electrodes on STN can be automatically estimated perioperatively.

If necessary, the clinician may then revise subcortical interface placement if the percentage of electrodes on target is estimated to be too low.

Also, electrodes from the cortical interface are used to evaluate the brain networks activated by subcortical stimulation on the target.

This gives valuable information on the cortical interface placement and the brain networks activated by delivered subcortical stimulation.

### Initial postoperative programming

After recovering from the surgery (typically 2 to 3 weeks postoperative), the system is ready to be activated and programmed to best suit the patient's specific PD symptoms.

Firstly, electrodes that are not on target must be identified and disregarded, and a reduced number of electrode clusters on target should be chosen as possible stimulating patterns, e.g. for the patient adapted treatment.

To achieve this automatically, neurophysiology measurements are performed while the subject is at rest and off medication. These measurements will be combined with perioperative measurements, cortical measurements, and imaging data, to reduce the number and the size of the chosen electrode clusters.

A pre-defined machine learning algorithm will integrate data from all inputs to refine the electrode selection. Then, the neurologist can evaluate the clinical effects of stimulation delivered from each of selected electrode clusters and set the therapeutic stimulation level that is best suited to the patient's need.

Finally, once the electrode cluster is selected with the chosen stimulation level set by the neurologist, network modulation is activated. This feature, in its basic operational mode at that stage, modulates the stimulation level (within therapeutic thresholds previously set) consistently with detection of symptomatic activity within the relevant brain network (identified with a combination of subcortical and cortical biomarkers).

The therapeutic effect of basal ganglia stimulation is associated to activation of brain network at different levels **[19]** with activation of specific brain areas **[20].**

The intended mode of operation of the system is to capture and use this brain network activation to adapt the stimulation delivered at basal ganglia level.

Such a feature allows the system to normalize the signatures of widespread brain network connectivity towards those found in healthy controls **[19].**

A schematic view illustrating the functioning of an open-loop DBS system (**left panel**), a closed-loop system using subcortical LFP signals (**middle panel**), and a closed-loop system using a combination of subcortical and cortical recordings to regulate brain network response with subcortical high selectivity stimulation (**right panel**), is provided in **Fig. 12** **[62].**

### Therapeutic learning

Following the initial programming, the patient can go home and benefit from therapeutic optimization, which will now be adjusted in real-time in accordance with parameters and boundaries set by the clinician.

This corresponds to the basic intended therapeutic use of the system and a significant reduction of symptoms is expected with limited side effects.

Data collection will further optimize and personalize programming for better symptoms targeting and improved clinical benefits.

During this learning phase, there is a continuous wideband recording of neural signals sequentially on all available electrodes during the therapy.

Random stimulation patterns (combination of electrodes chosen among those defined to be on target) will be delivered within safety limits to evaluate the behavior of biomarkers for learning purposes.

Patient direct inputs (e.g., through a dedicated application running on the patient computer) are also expected, e.g., to inform on symptoms type and magnitude, or drug intake.

Because of battery capacity and volume of data constraints, this learning phase can only occur at given moment during the day. During these recordings, extra usability constraints can be applied for power management (e.g., keep the external charger on for long period of time), for data storage (e.g., open the patient app on phone), or for the patient to give information on symptoms or state.

This phase will be facilitated by specific motor tasks (e.g., finger tapping, balance exercise, shape drawing, rest) conducted from the patient application to trigger particular recording sessions. This learning phase will be executed after first postoperative programming, or anytime a few days prior to a follow-up programming visit.

Analyzing neurophysiological signals coming from the system (and additionally also from other non-implantable wearable devices) is essential to set and control in real-time the optimal subcortical therapeutic modulation patterns and parameters, and get full benefit of high-density modulation and multiple decoding streams.

Due to physical constraints (battery size, memory space, data exchange rates, or the like), most of the computation steps must be performed offline, and machine learning algorithms should be trained to generalize to a large population of patients and adapt to each individual.

The system will then embed a diagnosis and evaluation software designed to provide physiological and therapeutic metrics and an Al-based software assisting clinicians in system programming. The main function of this feature will be to help clinicians interpreting biological data and decide on the best therapy for each patient.

Neural prostheses (such as the system of the invention), capable of multi-channel neural sensing, on-site signal processing, rapid symptom detection, and closed loop stimulation, are critical in order to further improve neuromodulation therapies in PD subjects.

Integration of disease biomarkers and machine learning algorithms could enable real-time and on-site processing of neural activity with no need for power-demanding telemetry.

To achieve this, the system will combine artificial intelligence (machine and deep learning) with high resolution neural interfaces and decoding for treatment of PD symptoms.

Moreover, deep learning will enable personalized detection of symptoms-specific biomarkers to potentially adjust stimulation parameters to reduce accessible motor and non-motor symptoms selectively.

However, deep learning algorithms are energy demanding and will be generated offline, to speed up their migration to real-time algorithms that can run on the system.

### Follow-up programming

During the learning phase, the clinician accesses patient data that has been collected.

Adjustments of the programming parameters, particularly on the closed-loop parameters, are proposed by the system.

Data collected during the learning phase are processed by the machine learning algorithm.

Adjusted stimulation patterns, biomarkers, and closed-loop parameters are proposed as an optimization.

This optimization can be related to stimulation levels, to stimulation patterns (i.e., a cluster of energized electrodes), but also to symptom-specific selective stimulation.

If online access to data is possible, this phase can occur before the patient's visit.

During the onsite programming session, the clinician can then apply the adjusted system programming as proposed by the algorithm and then evaluate its effective benefits with the patient.

### Home use

The patient benefits from personalized neuromodulation therapy, with expected maximized clinical benefits.

The patient can use the patient computer to follow-up and visualize the medical outcomes of the delivered stimulation therapy, evolution of drug intake, mood, and symptoms.

Follow-up with the clinician can also be planned through the patient computer.

### Pre-clinical testing

To evaluate stimulation and sensing capabilities of the neural interfaces according to the invention, preclinical evaluations have been conducted by the inventors regarding rGO electrodes, also in comparison with the Platinum (Pt) electrodes according to the prior art.

The results of these investigations, conducted on representative prototypes, are included in the scientific paper Viana at al. **[24],** presently under revision.

Thin film devices were fabricated and the electrochemical performance and stability of 25 µm diameter rGO micro-electrodes were assessed in vitro in a phosphate buffer saline solution.

Electrochemical characterization of rGO is illustrated in **Figs. 13a-f [24].**

Cyclic voltammetry showed that between -0.9 and +0.9 V, rGO behaved capacitively and no significant faradaic reactions were identified (**Fig. 13a**). The cathodic and anodic charge storage capacitance of rGO averaged 33.7 and 27.6 mC/cm², respectively (N=3). Impedance spectroscopy demonstrated that the rGO electrodes exhibited an impedance of 40 kΩ (at 1 kHz) (**Fig. 13b**), which was an order of magnitude lower than that measured (245 kΩ at 1 kHz) for commercially available Platinum (Pt) electrodes of similar size (ø 60 µm, NeuroNexus).

To evaluate safety of stimulation using rGO electrodes, charge injection limits and stimulation reliability tests were performed.

To achieve safe stimulation of the nervous tissue, the voltage drop at the electrode-electrolyte interface should always remain within the potential window set by the cyclic voltammetry in which no faradaic reactions occur.

**Fig. 13c** shows the potential excursion that a 25 µm diameter rGO microelectrode experiences upon the injection of rectangular, biphasic current pulses (1 ms/phase) at charge densities of 1, 2, 3, and 4 mC/cm².

A quasi-linear voltage shift of maximum 0.9 V exists between the ohmic drops at the edges of the pulses, indicating a capacitive-like behavior of the electrode-electrolyte interface within the limits of the potential window.

The charge injection limit of rGO electrodes was found to be 2.75 mC/cm² for 1-ms pulses.

**Fig. 13d** shows a map of the voltage polarization experienced by a rGO microelectrode in response to biphasic current pulses between 0.1 ms and 1 ms and injected charge densities up to 5 mC/cm².

For short pulses of 0.1 ms, approximately 2 mC/cm² could be safely injected since the volumetric capacitance of the electrode could not be fully charged.

The charge injection limit of rGO is 2 orders of magnitude higher than that of Pt electrodes, which is typically in the range of 0.02-0.04 mC/cm².

Electrode stability was investigated during continuous electrical stimulation. rGO were stimulated with 1 ms biphasic (1 ms interphase gap), 3 mC/cm² pulses at 100 Hz while the impedance and CSC were monitored every 500k pulses. rGO was observed to be stable after 15 million pulses as indicated by the consistent impedance at 1 kHz (**Fig. 13e**).

The average charge storage capacitance increased from 61.7 to 136.7 mC/cm² throughout the stimulation (**Fig. 13f**).

No structural changes were observed in the electrode.

The high performance and stability provided by rGO are attributed to the combination of the capacitive interaction in aqueous media with the large electrochemical surface area obtained during the hydrothermal reduction **[24].**

Sensing capabilities of rGO in the context of a laboratory experiment carried out on rodent models are illustrated in **Figs. 14a-f [24].**

Here, rGO electrode arrays were used to demonstrate ability to detect cortical brain signals with high accuracy and with better performance than known Pt/lr electrodes by implanting thin film with rGO electrodes and by recording neural activity at the surface of the auditory cortex of anesthetized rats (**Figs 14a-f)**.

The used system included an array of 64 rGO microelectrodes (ø 25 µm, pitch 300µm) that was positioned over the rat auditory cortex.

**Fig. 14b** shows the 10 Hz high-pass filtered signal from each of the 64 rGO electrodes in the array over a 350 ms window in which a pure tone was presented acoustically at the ear of the animal.

Large negative and positive voltage peaks were observed after the onset and offset of the sound stimulus indicating the recording of evoked local field potentials (LFP).

The density of the array allowed mapping of single-event LFPs as displayed in **Fig. 14b,** which reveals a spatial clustering with ON responses located on the top right corner and OFF responses grouped in the bottom part of the array.

The amplitudes of the evoked LFPs ranged between 500 µV and 700 µV (**Fig. 14c**).

High-pass filtered signals also showed the detection of high frequency multi-unit activity and the corresponding increase of the root mean square (RMS) amplitude (due to multi-unit activity) correlated to the evoked LFPs (**Fig. 14c**), indicating synchronous behavior of the neurons to the stimulus.

The signal to noise ratio was higher (2-3 times) and the noise floor was significantly lower, even for micro-size rGO electrodes (25-50 um diameter) that enable detection of high frequency signals.

The small dimension of rGO electrodes allows obtaining high-density arrays for sensing with improved decoding capabilities compared to low-density, larger Pt/lr electrodes.

Further, the incorporation of different electrodes with different size in the rGO arrays demonstrated the ability to detect a larger range of frequencies, from low (<50 Hz) to high (>150) Hz, with rGO outperforming Pt/lr electrodes at all frequency ranges.

Due to its increased charge injection capacity, rGO allows to generate precise stimulation with microelectrodes. This is relevant for focal, selective stimulation in small structures like the STN.

This has been demonstrated so far using peripheral nerve models: micro rGO electrodes implanted inside the sciatic nerve of rats (diameter of 0.8 mm) were able to achieve selective activation of the main nerve fascicles.

Focal stimulation with microelectrodes was able to achieve function specific targeted stimulation of submillimeter structures.

These data support the ability to control the selective activation of very fine structures using rGO microelectrodes.

The focal stimulation capability of the rGO electrodes is presently being tested in brain structures like the thalamus and STN in animals.

Ongoing studies in PD rodent models revealed the capability of small rGO electrodes to detect subcortical multi-unit spiking activity, paving the way to the identification of new PD symptomatic biomarkers, as illustrated in **Fig. 15****.**

In particular, **Fig. 15** shows inter-spike frequency distribution of multi-unit activity measured by small rGO electrode in the STN in healthy control rodents (see left panel), and in PD rodent model (see middle panel). Left panel shows the inter-spike frequency distribution in PD rodent model before (pre) and after (post) therapeutic STN stimulation.

Here, PD symptomatic activity reveals different inter-spike peak frequency profile than healthy controls, and this PD-specific profile tends to show similar profile as controls when therapeutic stimulation is provided in the STN.

### Conclusions

The properties of rGO electrodes enable the development of cortical and subcortical neural interfaces with a high density of small electrodes, and allow for selective and safe charge injection in conjunction with advanced signal sensing capabilities.

Such technology is critical for the development of a modulated stimulation system requiring focused selective stimulation for targeted therapy and high-resolution signal sensing for symptoms control and personalization.

### Supporting evidence

### High electrode density improves brain decoding

In addition to the currently approved leads shown in **Fig. 2** (see also **Fig. 16A,** reproducing the same leads shown in **Fig. 2** for comparison), other lead designs have been developed and are presently studied in the perspective of enhancing therapeutic outcome.

**Fig. 16B** shows examples of directional leads that have not yet been approved by the FDA, but have already been implanted in human patients for testing. These leads include the 8-electrode direct STNAcute lead and the 40-electrode Medtronic-Sapiens lead.

Also, **Fig. 16C** provides examples of additional lead designs that have not yet been implanted in human patients for testing, including the 16-electrode segmented lead discussed in Buhlmann et. al. **[25]** and the micro-array-based DBS (µDBS) lead with 1760 electrodes discussed in Willsie and Dorval **[26].**

Exemplary cross-sections of DBS electrodes according to the prior art are illustrated in **Fig. 16D** (in detail: **(i)** Medtronics 3387/ Medtronics 3389; **(ii)** 8-electrode Abbott Infinity^{®} directional lead, 8-electrode Boston Scientific directional lead, and STNAcute directional lead; **(iii)** Medtronic-Sapiens and 16-electrode lead discussed in Buhlmann et. al. **[25]; (iv)** example of µDBS lead, with 32 electrode per rotation).

Preliminary data regarding the use of relatively high-density subcortical leads has been recently collected and described.

For example, Medtronic-Sapiens subcortical lead (shown in **Fig. 16B**) includes 40 electrodes, arranged in 4 columns of 10-disc electrodes with dimensions of 0.66 mm x 0.8 mm (area = ca. 0.5 mm²). This lead provided about a 90° angular coverage with rhomboid combinations of 4 electrodes across three adjacent rows **[27].**

Using a similar experimental setup, simultaneous subthalamic LFPs at different depths and directions were recorded **[28].** These measurements were shown to provide confirmation of adequate lead placement and multidirectional spatial-temporal information potentially related to pathological subthalamic electrical activity and to the effect of stimulation.

Two similar leads have been used in pre-clinical experiments using a non-human primate model. In this context, the ability to modulate direction of stimulation **[33]** and to uncover heterogeneity of LFP activity within basal ganglia targets **[34], [35]** were demonstrated.

Acute intraoperative human studies leveraging subcortical electrode arrays suggested that oscillatory activities in the STN have a finer spatial resolution than what is detectable with commercially-available DBS leads **[28].**

Spectral and clinical topography of the STN on a large cohort of PD patients revealed that spectral features ranging from the delta-frequency band through HFOs are topographically segregated within the STN and may further differentiate in their spatial focality **[29]** as shown, e.g., in **Fig. 17** **[29].**

It was also observed that the projection of both the spectral and clinical stimulation hot spots into the STN revealed a varying degree of spatial overlap.

These results further support the need for small sensing electrodes to evaluate the different spectral features mapped within the STN, as well as small electrodes for selective hotspot stimulation.

In addition, small electrodes demonstrated the capability to provide not only higher spatial resolution, but also do sensing in a broader and wider range, in particular sense signals with larger frequency content. In line with theoretical predictions, small electrodes can detect signals that are otherwise lost or not detectable when sensing with electrodes of the size of those that are used in current clinical settings **[35].**

The subcortical neural interface according to the invention (**Fig. 8**), which includes a relatively high number of electrodes having a reduced area, and which further benefits from the properties of rGO, allows obtaining a higher precision in stimulation selectivity as well as a higher precision in the identification of signal biomarkers, when compared to the subcortical leads according to the prior art.

### Existing clinical evidence for increased benefits with smaller electrodes and focal stimulation

The use of commercially available directional leads having segmented electrodes (e.g. the 8-electrode Abbott Infinity^{®} directional lead or the 8-electrode Boston Scientific directional lead, **Figs. 2** and **16A**) revealed improvements in terms of therapeutic outcome in PD patients.

A double-blinded study evaluated intraoperatively the directional stimulation applied to STN in PD patients **[30].**

Here, the authors concluded that delivering directional stimulation to the STN in a "best direction" with a stimulating electrode of reduced size was able to improve the therapeutic window intraoperatively in comparison to omnidirectional. Additionally, 3D modeling revealed a reduced volume of activated tissue, suggesting a reduced likelihood of adverse stimulations **[30].**

These results have been confirmed by another randomized double-blinded trial, discussed in Dembek et al. **[36].**

Here, 20 monopolar reviews were conducted in 10 PD patients implanted in the STN with directional DBS in a prospective, randomized, double-blind design, to identify the best stimulation directions and compare them to conventional circular DBS regarding side-effect thresholds, motor improvement, and therapeutic window. In addition, circular and best-directional DBS were directly compared in a short-term crossover. Motor outcome was also assessed after an open-label follow-up of 3 to 6 months. Results confirmed postoperatively those from Pollo et. al. **[30],** demonstrating that stimulation in the individual best direction resulted in significantly larger therapeutic windows and higher side-effect thresholds than the case of circular DBS **[36].**

The blinded clinicians in charge of the programming also ranked the directional condition as the best option according to their clinical impressions. However, no difference in motor efficacy or stimulation amplitudes between directional and circular DBS in the short-term crossover was observed. Follow-up evaluations 3 to 6 months after implantation revealed improvements in terms of motor outcome and medication reduction, comparable to other DBS studies with a majority of patients remaining with a directional setting **[36].**

Also, recent studies confirmed that smaller electrodes at STN level were found to provide a larger therapeutic window **[30], [37], [38],** and a reduced volume of activated tissue **[37], [39]** when compared to omnidirectional stimulation delivered through larger ring electrodes. Finally, directional DBS was found to promote a significant reduction in disability over non-directional stimulation **[40],** and was preferred by blinded subjects and clinicians **[38].**

A box plot representing the width of the therapeutic window (percentiles 0, 25, 75, 100), for omnidirectional stimulation and for stimulation in each single direction is provided in **Fig. 18****,** left panel **[30].**

Also, a box plot of the electrical current producing a meaningful therapeutic effect (percentiles 0, 25, 75, 100), for omnidirectional stimulation and stimulation in each single direction is provided in **Fig. 18****,** right panel **[30].**

Data collected using investigational leads with high channel count further strengthens these results.

An acute intraoperative clinical study using the aforementioned investigational Medtronic-Sapiens lead was conducted in 8 PD patients. The results of this study are shown in **Figs. 19A-C** and discussed in Contarino et al **[31].**

In particular, the study revealed that the threshold could be increased by more than 1 mA while steering in at least one direction in comparison to conventional omnidirectional stimulation, thereby increasing the therapeutic window by up to 1.5 mA **[31].**

In particular, **Fig. 19C** shows that activation of an array of 4 electrodes yields a field of stimulation **(b)** which can be modulated so as to avoid stimulation of adjacent structures, thereby only stimulating the target area **[31].**

In summary, directional leads offer proof of principle evidence that directionality can result in an increase of benefit with a reduction in side effects. In addition, preliminary evidence with smaller electrodes suggested an even more precise control that can provide greater benefit. By the provision of a directional neural interface including smaller electrodes (**Figs. 7-8**), in combination with the additional benefits deriving from the use of rGO, the above-mentioned advantageous outcomes of known directional neural interfaces can be further improved.

### High electrode density allows to access different zones of the STN selectively

PD patients often benefit from DBS in terms of improved tremor, bradykinesia, rigidity, or motor fluctuations.

Instead, symptoms pertaining to speech, affect, cognition and even to the full range of motor features (e.g. freezing of gait) may persist, or even deteriorate, following surgery **[41].**

The limited resolution in stimulation of current DBS systems could explain this effect.

As argued in the foregoing, the STN has a complex organization with a dorsolateral motor area, a central cognitive/associative area, and a limbic medial tip. Also, each of these regions show dense innervation with many projections to other brain regions **[42],** suggesting that selective stimulation of STN could activate various networks within each area.

With current DBS lead designs (as shown, e.g., in **Figs. 2** and **16A),** using fairly large metal electrodes with limited resolution, access to such fine brain network selective activation is challenging.

However, at the surgical level, personalized targets within the STN to primarily address particular symptoms by stimulation of chosen brain networks were described in Hollunder et al. **[43].**

In particular, this personalized surgical targeting takes advantage of the identification of symptom-specific networks that have been investigated over the past years thanks to retrospective evaluation of DBS electrode location, connectomic network activation and clinical effects on corresponding symptoms across DBS cohorts, centers and disorders **[3], [19].**

In the approach described in Hollunder et al. **[43],** the placement of the DBS lead could be planned so that a set of cardinal symptoms could form the treatment focus, while additional comorbidities could be treated with lesser importance (i.e., weights could be applied based on empirical intensities of preoperatively experienced symptoms).

For example, preponderant tremor in one PD patient could shift the treatment focus toward maximized stimulation of the tremor network. By contrast, bradykinesia and rigidity symptoms may weigh more heavily in another PD patient, drawing particular focus onto modulating the corresponding circuitry.

Moreover, importance of side-effect networks may vary across individuals. While cognitive impairment or affective symptom networks should be avoided in all patients, their avoidance could receive even stronger emphasis in late-stage PD patients that already experience these symptoms.

Similarly, beneficial networks could not only be weighted based on empirical symptom severity, but also based on individual preferences. For instance, some patients may experience tremor as a stronger subjective burden than bradykinesia, or vice versa.

In a group of patients undergoing subthalamotomy, Rodriguez-Rojas et al. **[16]** could find a relationship between clinical outcomes after selective subthalamotomy and topography of the lesion.

As shown in **Fig. 20** **[16]** a clear spatial relationship could be found between STN lesion location within the motor area and the clinical effect on pathological symptoms.

The neural interface according to the invention (e.g. the cortical lead shown in **Fig. 8**), including an electrode array having small rGO electrodes with high charge injection capabilities, allows taking even more advantage of such an atlas of networks that, when modulated, clearly associate with different therapeutic effects.

**Fig. 21** provides a comparative diagram showing a representation of STN somatotopy as schematized by Sasaki et al. **[15]** with overlapped illustration of a known subcortical Boston Scientific directional DBS lead in an implanted state (left panel), and a subcortical neural interface (subcortical lead) according to the invention in an implanted state (right panel).

The subcortical neural interface of the invention (**Fig. 8**) allows obtaining a high level of spatial resolution that cannot be achieved through the lead designs according to the prior art, this resulting in an improved therapeutic benefit for the patient.

The neural interface according to the invention enables a more precise stimulation focus on the STN or GPi, with enhanced selectivity on the activated network.

Also, the large number of rGO micrometer-range electrodes allows selectively activating different parts of the STN independently. This enables getting precise access to specific networks associated with a particular symptom cluster, mapped on target area.

Such *symptomatotopy,* as introduced in Lozano and Lipsman **[44],** is accessible thanks to the high resolution and the high channel counts of the neural interface of the invention, as long as the neural interface has been well surgically placed within the STN.

The definition *symptomatotopy* means that stimulation-related symptoms relief is distributed and organized along the STN.

Another way to describe this concept is to assume, from a device point of view, each network that is selectively activated (with a cluster of small electrodes) as a therapeutic channel. Based on this assumption, the neural interface of the invention is capable to selectively activating distinct areas of the STN (and possibly multiple therapeutic channels), thereby offering the neurologist the possibility to independently address different symptoms with the delivered therapy.

Still further, the combination of subcortical and cortical interfaces as in the present invention allows for monitoring at cortical level of networks activated subcortically, which further improves stimulation resolution.

Due to the small size of the STN (with a volume of approximately 240 mm³), targeting motor areas coincides with an increased likelihood of hitting associative or limbic side-effect networks during optimization of the therapeutic effect.

Moreover, image-based electrode activation profile also shows limitations that are determined by tissue contrast, spatial resolution, and signal-to-noise ratio of currently available (clinical) imaging protocols.

Cortical monitoring of subcortical network activation, as implemented in the closed-loop system according to the invention, is therefore helpful in order to counteract the limitations of the known solutions.

In particular, the combination of a high-resolution subcortical neural interface (Fig. 8) and a high-resolution cortical neural interface (**Fig. 7**) for sensing brain signals enables the neurologist to prescribe and control stimulation therapy for multiple specific observed symptoms.

As a result, multiple motor and non-motor symptoms can be reduced on demand, this being particularly advantageous considering that neurological disorders such as PD usually show a large variety of symptoms fluctuating in time, together with the evolutive nature of the disease.

### Evidence and limitations of LFP-driven closed-loop (Adaptive) DBS

The advantages offered by closed-loop stimulation over conventional open-loop stimulation are apparent from evidence collected using the Medtronic PC+S (Percept^{®}) platform.

Swann et al. **[32]** reported a study on the use of the Medtronic Percept^{®} system in closed-loop configuration in two subjects. Here, , stimulation voltage was decreased when gamma oscillatory activity was high (indicating dyskinesia) and increased when gamma was low **[32].**

The experimental setup used in the study reported in Swann et al. **[32]** is schematically illustrated in **Fig. 22** **[32].**

In this proof-of-concept study conducted in 2 subjects, increased clinical benefits could not be demonstrated, but were shown to be maintained with much lower power budget (by about 40%) when compared to conventional open-loop approach. In fact, because adaptive DBS allows stopping the therapeutic stimulation when symptomatic LFP biomarkers are not detected, the power consumption can be significantly reduced, thereby increasing battery life with less charging and reduced battery replacement surgery in time for patients.

Clinical evidence suggests that detection of signal biomarkers within the STN, for example beta-band peaks (beta-bands corresponds to the brain oscillations of electrical activity between 12.5 Hz and 30 Hz) **[45], [46]** in the recorded LFPs, may be used as triggers for onset and offset of stimulation.

Increased brain response synchronization, measured as increased beta-band power spectrum, is observed within the STN of PD patients and correlates with disease symptoms. This can also be reduced through Levodopa treatment **[47], [48].**

Therapeutic DBS has proven effective in reducing such anomalous network synchronization (i.e., reduction of beta-band power spectrum).

Adaptive DBS could therefore take advantage of such signals, in particular by initiating stimulation when those signals detected, and interrupt stimulation when those signals are suppressed, in a closed-loop manner **[46].**

Further, detection of LFPs may allow to select optimal stimulation electrode based on biomarker recording localization (e.g., highest beta-band power).

Despite extensive research in that area, adaptive DBS stimulation with beta bands only partially considers the symptoms BNND associated with PD. In fact, the beta band frequency range (approx. 13-35 Hz) is modulated by many other normal functions of the brain (including movement or even planning or imagination of it) wakefulness and decision making. Therefore, despite established reports of its modulations in PD, the multifaceted involvement of beta band in physiological processes might affect its performance as a biomarker by itself for the diseased state, especially in the freely behaving patients (in an ecological setting rather than in a controlled clinical setting) with a chronic DBS implant **[49].**

Adaptive DBS only using beta bands is expected to reduce stimulation-induced side effects by limiting DBS stimulation to moments when it is needed. However, if patient tolerability (and, consequently, quality of life) is hypothesized to be improved, such adaptive stimulation is not intended to have any influence on therapeutic action and symptoms control.

An implementation of such adaptive DBS method in a non-human primate PD model is discussed in Johnson et al. **[50].** Here, the results revealed a reduction of on-time stimulation by about 50% (suggesting improved battery life) when compared to standard "always on" DBS.

However, no significant difference on symptoms control was detected.

The authors suggested to consider other biomarkers but beta bands in the STN as a feedback control, as well as different biomarkers in different locations, with the purpose of monitoring physiological activity throughout the basal ganglia thalamo-cortical circuit.

Adaptive DBS only using beta bands, which is not yet clinically approved and therefore not implemented in the context of the clinical routine, is mainly aimed at reducing the patient risk profile of DBS as well as optimizing the existing approach. However, it still does not constitute a change of paradigm in brain neuromodulation therapy for motor dysfunctions as the core therapeutic mechanism of action (broad electric stimulation of deep structure without specific brain network response control) remains unchanged.

Moreover, using only beta band oscillations at STN level for closing the loop may potentially affect the overall clinical benefit as this biomarker is influenced by physiological brain functions and does not seem to fully inform about the symptoms.

The closed-loop system of the invention, integrating real-time neural activity monitoring and feedback allows significantly improving precision and effectiveness of the delivered subcortical stimulation therapy.

### The clinical interest in brain network-driven closed-loop

Measuring of the brain network activation to a subcortical stimulation with the implant system can be performed by introducing additional recording electrodes placed on other parts of the network.

In patients affected by neurological disorders such as PD and are treated with STN stimulation, additional sensing electrodes can be placed at the cortical level (e.g., the motor cortex, M1) or subcortically at the thalamus level (e.g., GPi, GPe) to sense cortical response to therapeutic subcortical stimulation, monitor symptomatic brain network activity, and/or decode specific movements or functions in relation to symptoms.

The invention, by combining cortical and subcortical neural interfaces, allows obtaining a more precise and targeted modulation of neural networks. In particular, cortical neural interfaces can decode specific neural signals related to motor control, but also sensory perception, and/or cognitive processes, allowing for real-time monitoring and feedback.

This information can then be used to optimize subcortical stimulation parameters and patterns.

The combination of cortical and subcortical neural interfaces revealed potential benefits in various applications.

In the field of movement disorders, as in the case of PD, cortical neural interfaces can decode neural signals associated with movement intention, enabling the closed-loop system to provide subcortical stimulation only when necessary.

This highly-adaptive approach provided by the invention allows improving symptom control as well as reducing side effects when compared with the solutions according to the prior art.

A first, major clinical interest in cortical sensing is to evaluate efficacy of delivered subcortical therapeutic stimulation.

STN stimulation has been demonstrated to elicit evoked responses in the sensorimotor cortex in PD patients **[51], [52].**

These evoked responses are consecutive to the stimulation of thalamo-cortical networks, as they are due to the antidromic activation of the hyperdirect pathway (short latency) as well as activation of other indirect pathways (long latencies).

Using this, the therapeutic effects of STN stimulation were then found to be measurable at the cortical level.

Indeed, in patients that are treated with DBS, the electrodes identified to lead to the best clinical effects were found to elicit strongest cortical evoked responses with shortest latencies **[51], [53].** Moreover, the shape and the amplitude of the observed cortical evoked responses may be linked to the level and the direction of the applied stimulation **[54],** suggesting that cortical-evoked responses may greatly facilitate the assessment of STN stimulation in terms of targeted areas, stimulation orientation and amplitude.

Also, beyond STN beta activity, other symptomatic PD signals could be sensed at the cortical level, giving a brain network impairment perspective in such biomarker. For instance, it has been observed that, in PD, basal ganglia disease produces abnormal coupling between the phase of the beta-band rhythm and the amplitude of broadband gamma-band activity in motor controlling cortical areas **[55].**

Such phase abnormal coupling was observed to be reduced by therapeutic DBS **[56],** and could then be used as a specific brain network related symptomatic biomarker of PD **[57].** Still further, experimental setups combining existing DBS techniques with cortical sensing demonstrated significant benefits regarding symptoms decoding and control.

For instance, electrocorticography (ECoG) from cortical strip electrodes placed to the hand knob motor cortex region was found to show superior accuracy for movement decoding (grip force decoding) than LFPs recorded in the STN **[58],** supporting the utility of additional ECoG for improved adaptive DBS for PD patients.

Moreover, based on brain connectivity map, it could be predicted how well a specific recording site would perform to decode grip force.

Such a framework (termed prediction network mapping) can be used in the system of the invention to identify connectomic networks from which brain sensing is enabled to predict symptoms and behavior.

In patients suffering from essential tremor treated with DBS, the stimulation amplitude was adapted based on patient-specific motor behavior, suppressing the pathological tremor on-demand based on a cortical lead detecting upper limb motor activity **[59].**

Such stimulation paradigm was able to achieve clinical efficacy and tremor suppression in a range of movements (such as cup reaching, proximal and distal posture, water pouring, and/or writing) while having a consistent reduction in energy delivery (this reducing DBS-induced side effects) in comparison with standard DBS. In a similar concept, an experimental setup with direct recording of potentials in the primary motor cortex using subdural ECoG together with synchronous capture of gait freezing using optoelectronic motion-tracking systems in freely-walking patients with PD receiving STN DBS was implemented **[60].**

This study allowed to identify a particular physiological signature for freezing of gait in the ECoG measures (high beta-gamma phase-amplitude coupling in the primary motor cortex). This biomarker was then successfully used to control the subcortical DBS with significant improvement of freezing of gait, known to be difficult to treat with existing DBS.

In summary, this knowledge suggests that, firstly, cortical recordings are related to the clinical efficacy of the subcortical stimulation, which may greatly ease device programming and stimulation optimization.

Secondly, symptomatic biomarkers sensed cortically, reflecting BNND at upper brain network levels, were shown to be suppressed with subcortical stimulation, suggesting the possibility to regulate brain network response with subcortical stimulation and cortical recording.

Lastly, cortical sensed neural activity were observed to give a precise estimation of the symptoms' nature and onset, which can then be used to control stimulation of subcortical ganglia to efficiently address these symptoms only when needed.

However, the solutions that are presently available are still unable to precisely deliver stimulation only when required, addressing BNND with regulation of the network response with expected mitigation of subcortical stimulation-induced side effects.

The neural interface and closed-loop system of the invention addresses the above-mentioned drawbacks of the prior art, in particular by providing a solution that allows improving the decoding accuracy of cortical interfaces, optimizing stimulation parameters, and better understanding the complex interactions within neural networks.

In particular, thanks to the use of high-resolution, graphene-based neural interfaces, the closed-loop system of the invention allows improving decoding accuracy and providing more targeted and selective stimulation.

In summary, the closed-loop system of the invention, relying on a combination of cortical and subcortical neural interfaces, offers a powerful approach to modulate neural networks that overcomes the limitations of the prior art.

In particular, the combined approach integrating real-time neural activity monitoring and feedback allows enhancing precision and effectiveness of subcortical therapeutic stimulation, thereby improving therapeutic outcome.

It should be noted that the example control and estimation routines included herein can be used with various system configurations. The control methods and routines disclosed herein may be stored as executable instructions in non-transitory memory and may be carried out by a control unit such as a microcontroller (or a computer) in combination with the components of the system. The specific routines described herein may represent one or more of any number of processing strategies such as event-driven, interrupt-driven, multi-tasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of a computer readable storage medium in a control unit (e.g. a microcontroller) of the system, where the described actions are carried out by executing the instructions in a system including the various hardware components in combination with an electronic control unit.

### Bibliography

**[1]** GBD 2016 Parkinson's Disease Collaborators, (2018). Global, regional, and national burden of Parkinson's disease, 1990-2016: a systematic analysis for the Global Burden of Disease Study 2016. The Lancet. Neurology, 17(11), 939-953. https://doi.org/10.1016/S1474-4422(18)30295-3
**[2]** Kim, J., Criaud, M., Cho, S. S., Diez-Cirarda, M., Mihaescu, A., Coakeley, S., Ghadery, C., Valli, M., Jacobs, M. F., Houle, S., & Strafella, A. P. (2017). Abnormal intrinsic brain functional network dynamics in Parkinson's disease. Brain. A Journal of Neurology, 140(11), 2955-2967. https://doi.org/10.1093/brain/awx233.
**[3]** Horn, A., Wenzel, G., Irmen, F., Huebl, J., Li, N., Neumann, W.-J., Krause, P., Bohner, G., Scheel, M., & Kühn, A. A. (2019). Deep brain stimulation induced normalization of the human functional connectome in Parkinson's disease. Brain, 142(10), 3129-3143. https://doi.org/10.1093/brain/awz239
**[4]** Husain, M. (2019). Targeting network dysfunction in neurodegenerative diseases. Brain, 142(12), 3661-3662. https://doi.org/10.1093/brain/awz347
**[5]** Chou, K. L. (2008). Adverse events from the treatment of Parkinson's disease. Neurologic Clinics, 26(3 Suppl), S65-83, vi. https://doi.org/10.1016/j.ncl.2008.05.003
**[6]** Williams, N. R., Foote, K. D., & Okun, M. S. (2014). STN vs. GPi Deep Brain Stimulation: Translating the Rematch into Clinical Practice. Movement Disorders Clinical Practice, 1(1), 24-35. https://doi.org/10.1002/mdc3.12004
**[7]** Bratsos, S. P., Karponis, D., & Saleh, S. N. (2018). Efficacy and Safety of Deep Brain Stimulation in the Treatment of Parkinson's Disease: A Systematic Review and Meta-analysis of Randomized Controlled Trials. Cureus. https://doi.org/10.7759/cureus.3474
**[8]** Vizcarra, J. A., Situ-Kcomt, M., Artusi, C. A., Duker, A. P., Lopiano, L., Okun, M. S., Espay, A. J., & Merola, A. (2019). Subthalamic deep brain stimulation and levodopa in Parkinson's disease: a meta-analysis of combined effects. Journal of Neurology, 266(2), 289-297. https://doi.org/10.1007/s00415-018-8936-2
**[9]** Vitek, J. L., Jain, R., Chen, L., Tröster, A. I., Schrock, L. E., House, P. A., Giroux, M. L., Hebb, A. O., Farris, S. M., Whiting, D. M., Leichliter, T. A., Ostrem, J. L., San Luciano, M., Galifianakis, N., Verhagen Metman, L., Sani, S., Karl, J. A., Siddiqui, M. S., Tatter, S. B., ... Starr, P. A. (2020). Subthalamic nucleus deep brain stimulation with a multiple independent constant current-controlled device in Parkinson's disease (INTREPID): a multicentre, double-blind, randomised, sham-controlled study. The Lancet. Neurology, 19(6), 491-501. https://doi.org/10.1016/S1474-4422(20)30108-3
**[10]** Lyons, K. E., & Pahwa, R. (2006). Effects of bilateral subthalamic nucleus stimulation on sleep, daytime sleepiness, and early morning dystonia in patients with Parkinson disease. Journal of Neurosurgery, 104(4), 502-505. https://doi.org/10.3171/jns.2006.104.4.502
**[11]** Appleby, B. S., Duggan, P. S., Regenberg, A., & Rabins, P. V. (2007). Psychiatric and neuropsychiatric adverse events associated with deep brain stimulation: A meta-analysis of ten years' experience. Movement Disorders: Official Journal of the Movement Disorder Society, 22(12), 1722-1728. https://doi.org/10.1002/mds.21551
**[12]** Rasiah, N. P., Maheshwary, R., Kwon, C.-S., Bloomstein, J. D., & Girgis, F. (2023). Complications of Deep Brain Stimulation for Parkinson Disease and Relationship between Micro-electrode tracks and hemorrhage: Systematic Review and Meta-Analysis. World Neurosurgery, 171, e8-e23. https://doi.org/10.1016/j.wneu.2022.10.034
**[13]** Wang, J. T., Wang, A. Y., Psarros, C., & Da Cruz, M. (2014). Rates of revision and device failure in cochlear implant surgery: a 30-year experience. The Laryngoscope, 124(10), 2393-2399. https://doi.org/10.1002/lary.24649
**[14]** Plantinga, B. R., Temel, Y., Duchin, Y., Uludaǧ, K., Patriat, R., Roebroeck, A., Kuijf, M., Jahanshahi, A., Ter Haar Romenij, B., Vitek, J., & Harel, N. (2018). Individualized parcellation of the subthalamic nucleus in patients with Parkinson's disease with 7T MRI. Neurolmage, 168, 403-411. https://doi.org/10.1016/j.neuroimage.2016.09.023
**[15]** Sasaki, T., Kuwahara, K., Kin, I., Okazaki, M., Sasada, S., Shinko, A., Kameda, M., Yasuhara, T., Agari, T., & Date, I. (2019). Identification of Somatotopic Organization and Optimal Stimulation Site Within the Subthalamic Nucleus for Parkinson's Disease. Operative Neurosurgery (Hagerstown, Md.), 17(3), 239-246. https://doi.org/10.1093/ons/opy351
**[16]** Rodriguez-Rojas, R., Pineda-Pardo, J. A., Mañez-Miro, J., Sanchez-Turel, A., Martinez-Fernandez, R., del Alamo, M., DeLong, M., & Obeso, J. A. (2022). Functional Topography of the Human Subthalamic Nucleus: Relevance for Subthalamotomy in Parkinson's Disease. Movement Disorders, 37(2), 279-290. https://doi.org/10.1002/mds.28862
**[17]** Ineichen, C., Shepherd, N. R., & Sürücü, O. (2018). Understanding the Effects and Adverse Reactions of Deep Brain Stimulation: Is It Time for a Paradigm Shift Toward a Focus on Heterogenous Biophysical Tissue Properties Instead of Electrode Design Only? Frontiers in Human Neuroscience, 12, 468. https://doi.org/10.3389/fnhum.2018.00468
**[18]** Powers, R., Etezadi-Amoli, M., Arnold, E. M., Kianian, S., Mance, I., Gibiansky, M., Trietsch, D., Alvarado, A. S., Kretlow, J. D., Herrington, T. M., Brillman, S., Huang, N., Lin, P. T., Pham, H. A., & Ullal, A. V. (2021). Smartwatch inertial sensors continuously monitor real-world motor fluctuations in Parkinson's disease. Science Translational Medicine, 13(579). https://doi.org/10.1126/scitranslmed.abd7865
**[19]** Horn, A., Reich, M., Vorwerk, J., Li, N., Wenzel, G., Fang, Q., Schmitz-Hübsch, T., Nickl, R., Kupsch, A., Volkmann, J., Kühn, A. A., & Fox, M. D. (2017). Connectivity Predicts deep brain stimulation outcome in Parkinson disease. Annals of Neurology, 82(1), 67-78. https://doi.org/10.1002/ana.24974
**[20]** Boutet, A., Madhavan, R., Elias, G. J. B., Joel, S. E., Gramer, R., Ranjan, M., Paramanandam, V., Xu, D., Germann, J., Loh, A., Kalia, S. K., Hodaie, M., Li, B., Prasad, S., Coblentz, A., Munhoz, R. P., Ashe, J., Kucharczyk, W., Fasano, A., & Lozano, A. M. (2021). Predicting optimal deep brain stimulation parameters for Parkinson's disease using functional MRI and machine learning. Nature Communications, 12(1), 3043.
**[21]** Aman, J. E., Johnson, L. A., Sanabria, D. E., Wang, J., Patriat, R., Hill, M., Marshall, E., MacKinnon, C. D., Cooper, S. E., Schrock, L. E., Park, M. C., Harel, N., & Vitek, J. L. (2020). Directional deep brain stimulation leads reveal spatially distinct oscillatory activity in the globus pallidus internus of Parkinson's disease patients. Neurobiology of Disease, 139, 104819. https://doi.org/10.1016/j.nbd.2020.104819
**[22]** Telkes, I., Sabourin, S., Durphy, J., Adam, O., Sukul, V., Raviv, N., Staudt, M. D., & Pilitsis, J. G. (2020). Functional Use of Directional Local Field Potentials in the Subthalamic Nucleus Deep Brain Stimulation. Frontiers in Human Neuroscience, 14, 145. https://doi.org/10.3389/fnhum.2020.00145
**[23]** Shen, L., Jiang, C., Hubbard, C. S., Ren, J., He, C., Wang, D., Dahmani, L., Guo, Y., Liu, Y., Xu, S., Meng, F., Zhang, J., Liu, H., & Li, L. (2020). Subthalamic Nucleus Deep Brain Stimulation Modulates 2 Distinct Neurocircuits. Annals of Neurology, 88(6), 1178-1193. https://doi.org/10.1002/ana.25906
**[24]** Viana, D., Walston, S. T., Illa, X., del Valle, J., Prats, E., de la Oliva, N., Palma, M., del Corro, E., Rodriguez-Meana, B., del Pilar Bernicola, M., Rodriguez-Lucas, E., Gener, T. A., de la Cruz, J. M., Torres-Miranda, M., Sperling, J., Martí-Sánchez, S., Spadaro, M. C., Hébert, C., Arbiol, J., ... Garrido, J. A. (n.d.). Graphene-based thin film microelectrode technology for in vivo high resolution neural recording and stimulation. Under Review at Advanced Materials.
**[25]** Buhlmann, J., Hofmann, L., Tass, P. A., & Hauptmann, C. (2011). Modeling of a segmented electrode for desynchronizing deep brain stimulation. Frontiers in Neuroengineering, 4, 15. https://doi.org/10.3389/fneng.2011.00015
[26] Willsie, A., & Dorval, A. (2015). Fabrication and initial testing of the µDBS: a novel Deep Brain Stimulation electrode with thousands of individually controllable contacts. Biomedical Microdevices, 17(3), 9961. https://doi.org/10.1007/s10544-015-9961-x
[27] Anderson, D. N., Osting, B., Vorwerk, J., Dorval, A. D., & Butson, C. R. (2018). Optimized programming algorithm for cylindrical and directional deep brain stimulation electrodes. Journal of Neural Engineering, 15(2), 026005. https://doi.org/10.1088/1741-2552/aaa14b
[28] Bour, L. J., Lourens, M. A. J., Verhagen, R., de Bie, R. M. A., van den Munckhof, P., Schuurman, P. R., & Contarino, M. F. (2015). Directional Recording of Subthalamic Spectral Power Densities in Parkinson's Disease and the Effect of Steering Deep Brain Stimulation. Brain Stimulation, 8(4), 730-741. https://doi.org/10.1016/j.brs.2015.02.002
[29] Averna, A., Debove, I., Nowacki, A., Peterman, K., Duchet, B., Sousa, M., Bernasconi, E., Alva, L., Lachenmayer, M. L., Schuepbach, M., Pollo, C., Krack, P., Nguyen, T.-A. K., & Tinkhauser, G. (2023). Spectral Topography of the Subthalamic Nucleus to Inform Next-Generation Deep Brain Stimulation. Movement Disorders: Official Journal of the Movement Disorder Society, 38(5), 818-830. https://doi.org/10.1002/mds.29381
[30] Pollo, C., Kaelin-Lang, A., Oertel, M. F., Stieglitz, L., Taub, E., Fuhr, P., Lozano, A. M., Raabe, A., & Schüpbach, M. (2014). Directional deep brain stimulation: an intraoperative double-blind pilot study. Brain A Journal of Neurology, 137(Pt 7), 2015-2026. https://doi.org/10.1093/brain/awu102
[31] Contarino, M. F., Bour, L. J., Verhagen, R., Lourens, M. A. J., de Bie, R. M. A., van den Munckhof, P., & Schuurman, P. R. (2014). Directional steering: A novel approach to deep brain stimulation. Neurology, 83(13), 1163-1169. https://doi.org/10.1212/WNL.0000000000000823
[32] Swann, N. C., de Hemptinne, C., Thompson, M. C., Miocinovic, S., Miller, A. M., Gilron, R., Ostrem, J. L., Chizeck, H. J., & Starr, P. A. (2018). Adaptive deep brain stimulation for Parkinson's disease using motor cortex sensing. Journal of Neural Engineering, 15(4), 046006. https://doi.org/10.1088/1741-2552/aabc9b
[33] Martens, H. C. F., Toader, E., Decré, M. M. J., Anderson, D. J., Vetter, R., Kipke, D. R., Baker, K. B., Johnson, M. D., & Vitek, J. L. (2011). Spatial steering of deep brain stimulation volumes using a novel lead design. Clinical Neurophysiology Official Journal of the International Federation of Clinical Neurophysiology, 122(3), 558-566. https://doi.org/10.1016/j.clinph.2010.07.026
**[34]** Connolly, A. T., Vetter, R. J., Hetke, J. F., Teplitzky, B. A., Kipke, D. R., Pellinen, D. S., Anderson, D. J., Baker, K. B., Vitek, J. L., & Johnson, M. D. (2016). A Novel Lead Design for Modulation and Sensing of Deep Brain Structures. IEEE Transactions on Bio-Medical Engineering, 63(1), 148-157. https://doi.org/10.1109/TBME.2015.2492921
**[35]** Zhang, S., Connolly, A. T., Madden, L. R., Vitek, J. L., & Johnson, M. D. (2018). High-resolution local field potentials measured with deep brain stimulation arrays. Journal of Neural Engineering, 15(4), 046019. https://doi.org/10.1088/1741-2552/aabdf5
**[36]** Dembek, T. A., Reker, P., Visser-Vandewalle, V., Wirths, J., Treuer, H., Klehr, M., Roediger, J., Dafsari, H. S., Barbe, M. T., & Timmermann, L. (2017). Directional DBS increases side-effect thresholds-A prospective, double-blind trial. Movement Disorders Official Journal of the Movement Disorder Society, 32(10), 1380-1388. https://doi.org/10.1002/mds.27093
**[37]** Sabourin, S., Khazen, O., DiMarzio, M., Staudt, M. D., Williams, L., Gillogly, M., Durphy, J., Hanspal, E. K., Adam, O. R., & Pilitsis, J. G. (2020). Effect of Directional Deep Brain Stimulation on Sensory Thresholds in Parkinson's Disease. Frontiers in Human Neuroscience, 14, 217. https://doi.org/10.3389/fnhum.2020.00217
**[38]** Schnitzler, A., Mir, P., Brodsky, M. A., Verhagen, L., Groppa, S., Alvarez, R., Evans, A., Blazquez, M., Nagel, S., Pilitsis, J. G., Pötter-Nerger, M., Tse, W., Almeida, L., Tomycz, N., Jimenez-Shahed, J., Libionka, W., Carrillo, F., Hartmann, C. J., Groiss, S. J., ... Brodsky, M. A. (2022). Directional Deep Brain Stimulation for Parkinson's Disease: Results of an International Crossover Study With Randomized, Double-Blind Primary Endpoint. Neuromodulation: Technology at the Neural Interface, 25(6), 817-828. https://doi.org/10.1111/ner. 13407
**[39]** Nguyen, T. A. K., Djilas, M., Nowacki, A., Mercanzini, A., Schüpbach, M., Renaud, P., & Pollo, C. (2019). Analysis of patient-specific stimulation with segmented leads in the subthalamic nucleus. PloS One, 14(6), e0217985. https://doi.org/10.1371/journal.pone.0217985
**[40]** Rebelo, P., Green, A. L., Aziz, T. Z., Kent, A., Schafer, D., Venkatesan, L., & Cheeran, B. (2018). Thalamic Directional Deep Brain Stimulation for tremor: Spend less, get more. Brain Stimulation, 11(3), 600-606. https://doi.org/10.1016/j.brs.2017.12.015
**[41]** Rodriguez-Oroz, M. C., Moro, E., & Krack, P. (2012). Long-term outcomes of surgical therapies for Parkinson's disease. Movement Disorders, 27(14), 1718-1728. https://doi.org/10.1002/mds.25214
**[42]** Alkemade, A. (2013). Subdivisions and Anatomical Boundaries of the Subthalamic Nucleus. The Journal of Neuroscience, 33(22), 9233-9234. https://doi.org/10.1523/J N EU ROSCI .1266-13.2013
**[43]** Hollunder, B., Rajamani, N., Siddiqi, S. H., Finke, C., Kühn, A. A., Mayberg, H. S., Fox, M. D., Neudorfer, C., & Horn, A. (2022). Toward personalized medicine in connectomic deep brain stimulation. Progress in Neurobiology, 210, 102211. https://doi.org/10.1016/j.pneurobio.2021.102211
**[44]** Lozano, A. M., & Lipsman, N. (2013). Probing and Regulating Dysfunctional Circuits Using Deep Brain Stimulation. Neuron, 77(3), 406-424. https://doi.org/10.1016/j.neuron.2013.01.020
**[45]** Feldmann, L. K., Neumann, W.-J., Krause, P., Lofredi, R., Schneider, G.-H., & Kühn, A. A. (2021). Subthalamic beta band suppression reflects effective neuromodulation in chronic recordings. European Journal of Neurology, 28(7), 2372-2377. https://doi.org/10.1111/ene. 14801
**[46]** Neumann, W.-J., Degen, K., Schneider, G.-H., Brücke, C., Huebl, J., Brown, P., & Kühn, A. A. (2016). Subthalamic synchronized oscillatory activity correlates with motor impairment in patients with Parkinson's disease. Movement Disorders. Official Journal of the Movement Disorder Society, 31(11), 1748-1751. https://doi.org/10.1002/mds.26759
**[47]** Brown, P. (2003). Oscillatory nature of human basal ganglia activity: relationship to the pathophysiology of Parkinson's disease. Movement Disorders.: Official Journal of the Movement Disorder Society, 18(4), 357-363. https://doi.org/10.1002/mds.10358
**[48]** Neumann, W.-J., Staub-Bartelt, F., Horn, A., Schanda, J., Schneider, G.-H., Brown, P., & Kühn, A. A. (2017). Long term correlation of subthalamic beta band activity with motor impairment in patients with Parkinson's disease. Clinical Neurophysiology. Official Journal of the International Federation of Clinical Neurophysiology, 128(11), 2286-2291. https://doi.org/10.1016/j.clinph.2017.08.028
**[49]** Ozturk, M., Viswanathan, A., Sheth, S. A., & Ince, N. F. (2021). Electroceutically induced subthalamic high-frequency oscillations and evoked compound activity may explain the mechanism of therapeutic stimulation in Parkinson's disease. Communications Biology, 4(1). https://doi. org/10.1038/s42003-021-01915-7
**[50]** Johnson, L. A., Nebeck, S. D., Muralidharan, A., Johnson, M. D., Baker, K. B., & Vitek, J. L. (2016). Closed-Loop Deep Brain Stimulation Effects on Parkinsonian Motor Symptoms in a Non-Human Primate - Is Beta Enough? Brain Stimulation, 9(6), 892-896. https://doi.org/10.1016/j.brs.2016.06.051
**[51]** Kuriakose, R., Saha, U., Castillo, G., Udupa, K., Ni, Z., Gunraj, C., Mazzella, F., Hamani, C., Lang, A. E., Moro, E., Lozano, A. M., Hodaie, M., & Chen, R. (2010). The Nature and Time Course of Cortical Activation Following Subthalamic Stimulation in Parkinson's Disease. Cerebral Cortex, 20(8), 1926-1936. https://doi.org/10.1093/cercor/bhp269
**[52]** Li, Q., Qian, Z.-M., Arbuthnott, G. W., Ke, Y., & Yung, W.-H. (2014). Cortical Effects of Deep Brain Stimulation. JAMA Neurology, 71(1), 100. https://doi.org/10.1001/jamaneurol.2013.4221
**[53]** Walker, H. C., Huang, H., Gonzalez, C. L., Bryant, J. E., Killen, J., Cutter, G. R., Knowlton, R. C., Montgomery, E. B., Guthrie, B. L., & Watts, R. L. (2012). Short latency activation of cortex during clinically effective subthalamic deep brain stimulation for Parkinson's disease. Movement Disorders, 27(7), 864-873. https://doi.org/10.1002/mds.25025
**[54]** Peeters, J., Boogers, A., Van Bogaert, T., Davidoff, H., Gransier, R., Wouters, J., Nuttin, B., & Mc Laughlin, M. (2023). Electrophysiologic Evidence That Directional Deep Brain Stimulation Activates Distinct Neural Circuits in Patients With Parkinson Disease. Neuromodulation: Journal of the International Neuromodulation Society, 26(2), 403-413. https://doi.org/10.1016/j.neurom.2021.11.002
**[55]** de Hemptinne, C., Ryapolova-Webb, E. S., Air, E. L., Garcia, P. A., Miller, K. J., Ojemann, J. G., Ostrem, J. L., Galifianakis, N. B., & Starr, P. A. (2013). Exaggerated phase-amplitude coupling in the primary motor cortex in Parkinson disease. Proceedings of the National Academy of Sciences of the United States of America, 110(12), 4780-4785. https://doi.org/10.1073/pnas.1214546110
**[56]** de Hemptinne, C., Swann, N. C., Ostrem, J. L., Ryapolova-Webb, E. S., San Luciano, M., Galifianakis, N. B., & Starr, P. A. (2015). Therapeutic deep brain stimulation reduces cortical phase-amplitude coupling in Parkinson's disease. Nature Neuroscience, 18(5), 779-786. https://doi.org/10.1038/nn.3997
**[57]** Yu, Y., Han, F., & Wang, Q. (2022). Exploring phase-amplitude coupling from primary motor cortex-basal ganglia-thalamus network model. Neural Networks: The Official Journal of the International Neural Network Society, 153, 130-141. https://doi.org/10.1016/j.neunet.2022.05.027
[58] Merk, T., Peterson, V., Lipski, W. J., Blankertz, B., Turner, R. S., Li, N., Horn, A., Richardson, R. M., & Neumann, W.-J. (2022). Electrocorticography is superior to subthalamic local field potentials for movement decoding in Parkinson's disease. ELife, 11. https://doi.org/10.7554/eLife.75126
[59] Opri, E., Cernera, S., Molina, R., Eisinger, R. S., Cagle, J. N., Almeida, L., Denison, T., Okun, M. S., Foote, K. D., & Gunduz, A. (2020). Chronic embedded cortico-thalamic closed-loop deep brain stimulation for the treatment of essential tremor. Science Translational Medicine, 12(572).https://doi.org/10.1126/SCITRANSLMED.AAY7680
[60] Yin, Z., Zhu, G., Liu, Y., Zhao, B., Liu, D., Bai, Y., Zhang, Q., Shi, L., Feng, T., Yang, A., Liu, H., Meng, F., Neumann, W. J., Kühn, A. A., Jiang, Y., & Zhang, J. (2022). Cortical phase-amplitude coupling is key to the occurrence and treatment of freezing of gait. Brain: A Journal of Neurology, 145(7), 2407-2421. https://doi.org/10.1093/brain/awac121
[61] Bullmore, E., & Sporns, O. (2012). The economy of brain network organization. Nature Reviews Neuroscience, 13(5), 336-349. https://doi.org/10.1038/nrn3214
[62] Okun, Michael. Deep-Brain Stimulation - Entering the Era of Human Neural-Network Modulation 371, DOI 10.1056/NEJMp1408779 The New England journal of medicine

**Table 1 - Adverse events following DBS surgery in two recent studies (Appleby et al. [11] and Rasiah et al. [12])**

| Source | Number of subjects | Perioperative/postoperative hemorrhage (intracranial, cerebral) | Infection | Surgical site complications (e.g. seroma, skin erosion) | Device complication (e.g., Displacement, migration, explantation, hardware failure, lead fracture, battery failure...) |
|---|---|---|---|---|---|
| Appleby et. al., 2007 | 6573 | 135 (2%) | 1049 (16%) | 920 (14%) | 2204 (33.3%) |
| Rasiah et. al., 2022 | 21261 | 510 (2.4%) | 850 (4%) | 595 (2.8%) | 2252 (10.6%) |
| | **Total: 27834** | **645** | **1899** | **1515** | **4456** |
| | **Incidence** | **2.32%** | **6.82%** | **5.44%** | **16.01%** |

### References

- 100: Neural interface (cortical)
- 200: Neural interface (subcortical)
- 10, 20: Thin film
- 12, 22: Electrode array
- 14, 24: Electrode

- 300: Closed-loop system
- 30: Stimulation unit
- 32: Sensing unit
- 34: Control unit / implantable control unit
- 36: Extracorporeal power supply unit
- 38: Clinician computer
- 40: Display means (clinician computer)
- 42: Patient computer (wearable device)
- 44: User interface (display means)

- P: Patient
- S: Skull

## Claims

1. A neural interface (100, 200) for delivering neuromodulation/neurostimulation to brain structures of a patient (P) and/or sensing brain signals of the patient (P), the neural interface at least comprising:
a support (10, 20), wherein said support includes and/or carries at least an electrode array (12, 22),
wherein the electrode array comprises a plurality of electrodes (14, 24) made of reduced graphene oxide (rGO), each electrode (14, 24) of said plurality of electrodes (14, 24) having an area in the micrometer range.

2. The neural interface (100, 200) according to claim 1,
**characterized in that**
the area of each electrode (14, 24) is comprised between 1.0 and 0.0005 mm².

3. The neural interface (100, 200) according to claim 1 or 2,
**characterized in that**
the electrode array (12, 22) comprises at least 20 electrodes (14, 24),
preferably wherein the electrode array (12, 22) comprises at least 40 electrodes (14, 24),
preferably wherein the electrode array (12, 22) comprises at least 50 electrodes (14, 24),
preferably wherein the electrode array (12, 22) comprises between 128 and 1024 electrodes (14, 24).

4. The neural interface (100, 200) according to any one of the preceding claims,
**characterized in that**
the electrode array (12, 22) comprises a plurality of electrodes (14, 24) having different areas in the micrometer range.

5. The neural interface (100, 200) according to any one of the preceding claims,
**characterized in that**
said neural interface (100, 200) is configured and adapted to be implanted cortically or subcortically,
preferably wherein the neural interface (100, 200) is configured and adapted to deliver neuromodulation/neurostimulation and sensing brain signals of the patient (P).

6. The neural interface (100, 200) according to any one of the preceding claims,
**characterized in that**
said support (10, 20) at least partially is and/or comprises a thin film and/or is made of a nanostructure and/or a microstructure.

7. The neural interface (100, 200) according to claim 6,
**characterized in that**
the thin film (10, 20) is made of a flexible material,
preferably wherein said flexible material includes:
a metal or a metal alloy, and/or
a polymer material,
preferably wherein said polymer material includes one among Polylmide, Parylene, or Polydimethylsiloxane (PDMS).

8. A closed-loop system (300) for delivery of neuromodulation/neurostimulation to brain structures of a patient (P), comprising:
a stimulation unit (30) comprising at least one neural interface (100, 200) according to any one of claims 1 to 5, said at least one neural interface (100, 200) being configured and adapted to deliver neuromodulation/neurostimulation to brain structures of the patient (P);
a sensing unit (32) including at least one neural interface (100, 200) according to any one of claims 1 to 5, said at least one neural interface (100, 200) being configured and adapted to sense brain signals of the patient (P), and
a control unit (34), operatively connected to the stimulation unit (30) and the sensing unit (32), the control unit (34) being configured and adapted to perform real-time decoding of brain signals from the sensing unit (32) and control one or more stimulation parameters for the stimulation unit (30) based on said brain signals from the sensing unit (32).

9. The system (300) according to claim 8,
**characterized in that**
the at least one neural interface (200) of the stimulation unit (30) comprises a subcortical lead (200) and/or a subcortical lead (100),
preferably wherein said subcortical lead (200) and/or cortical lead (100) is configured and adapted to deliver selective stimulation to basal ganglia to elicit a therapeutic response.

10. The system (300) according to claim 8 or 9,
**characterized in that**
the sensing unit (32) includes at least one cortical neural interface (100) and/or at least one subcortical neural interface (200),
preferably wherein the sensing unit (32) includes one cortical neural interface (100) and one subcortical neural interface (200),
preferably wherein the brain signals include at least one among brain oscillation and/or evoked responses to subcortical stimulation.

11. The system (300) according to any one of claims 8 to 10,
**characterized in that**
the control unit (34) is configured and adapted to implement a machine learning algorithm for recording and processing brain signals of the patient (P) and generate stimulation patterns for the stimulation unit (30) based on the processed brain signals.

12. The system (300) according to any one of claims 8 to 11,
**characterized in that**
the control unit (34) is further configured and adapted to:
process brain signals from the sensing unit (32) and determine a physiological response of the patient (P) to the delivered stimulation therapy;
map subcortical stimulation target points based on the determined physiological response of the patient (P), and
control the stimulation unit (30) to deliver neuromodulation/neurostimulation based on the performed mapping.

13. The system (300) according to any one of claims 8 to 12,
**characterized in that**
the control unit (34) is an implantable control unit (34),
preferably wherein the control unit (34) is configured and adapted to be implanted on the patient's skull (S).

14. The system (300) according to claim 13,
**characterized in that**
the system (300) further comprises an extra-corporeal power supply unit (36) configured and adapted to wirelessly supply power to the implantable control unit (34).

15. The system (300) according to any one of claim 8 to 14,
**characterized in that**
the system (300) further comprises a clinician computer (38), said clinician computer (38) being configured and adapted to implement direct or indirect bi-directional communication with the control unit (34),
wherein the clinician computer (38) comprises a control means configured and adapted to run a dedicated software means to:
determine a patient's response to the delivered stimulation therapy based on detected brain signals from the sensing unit (32), and/or
provide an input signal to the control unit (34) to adjust one or more stimulation or control loop parameters for the stimulation unit based on the determined patient's response to the delivered therapy and/or one or more predefined, patient-specific therapeutic targets.

16. The system (300) according to claim 15,
**characterized in that**
said software means of the clinician computer (38) is configured and adapted to implement web or cloud-based computing and/or machine learning algorithms and/or deep learning algorithms to:
analyze detected brain signals of the patient (P);
define the patient's response to the delivered therapy based on said detected brain signals, and
define optimized stimulation parameters based on the determined patient's response to the delivered stimulation therapy,
preferably wherein the patient's response to the delivered stimulation therapy is defined based on personalization algorithm suggestions, more preferably AI-based personalization algorithm suggestions.

17. The system (300) according to any one of claims 8 to 16,
**characterized in that**
the clinician computer (38) further comprises a display means (40) for displaying information regarding the detected brain signals, current stimulation parameters and/or other information on the delivered stimulation therapy.

18. The system (300) according to any one of claims 15 to 17,
**characterized in that**
said software means of the clinician computer (38) is further configured and adapted to:
collect and store data regarding the patient's response to the delivered stimulation therapy for a plurality of patients (P) subject to treatment, and
classify each of said patients (P) based on a respective response to the delivered stimulation therapy,
preferably wherein said software means is configured and adapted to classify patients (P) according to a respective health-risk level, in particular according to the following ranking criteria:
low risk;
medium risk;
high risk.

19. The system (300) according to any one of claim 8 to 18,
**characterized in that**
the system (300) further includes a patient computer (42), said patient computer (42) being configured and adapted to implement direct or indirect bi-directional communication with the control unit (32),
wherein the patient computer (42) comprises a control means configured and adapted to run a dedicated software means to:
receive a user input though a user input means of the patient computer (42), and
provide an input signal to the control unit (34) to adjust one or more stimulation or control loop parameters for the stimulation unit (30), based on said user input,
preferably wherein said user input means includes a display means (44) of the touch-screen type.

20. The system (300) according to claim 19,
**characterized in that**
the patient computer (42) is further configured and adapted to implement direct or indirect bi-directional communication with the clinician computer (38),
wherein the control means of the patient computer is further configured and adapted to:
receive a feedback from the patient (P) regarding the delivered stimulation therapy, provided through said user input means, and
forwarding the patient's feedback to the clinician computer (38) in real time.

21. The system (300) according to any one of claim 19 or 20,
**characterized in that**
the patient computer (42) is a wearable device capable of implementing smart functionalities,
preferably wherein said wearable device is provided with a sensor means that is configured and adapted to measure physiological data of the patient,
preferably wherein said physiological data of the patient include one or more among heart rate, blood pressure, body temperature and/or body motion/acceleration.
